# EUROPEAN PATENT SPECIFICATION

(11) **EP 2 271 358 B1**
(45) Date of publication and mention of the grant of the patent: **21.11.2018**
(21) Application number: 09724166.5
(22) Date of filing: 25.03.2009
(51) Int. Cl.: A61K 35/30, A61K 9/00, A61P 25/16, A61K 38/18

(54) **use of mesencephalic astrocyte-derived neurotrophic factor for treating Parkinson's disease**
verwendung von mesencephalischem von Astrozyten abstammenden neurotrophen Faktor zur Behandlung von Parkinsonscher Erkrankung
utilisation d'un facteur neurotrophique dérivé des astrocytes pour le traitement de la maladie de Parkinson

(30) Priority: 25.03.2008 US 39334 P
(43) Date of publication of application: 12.01.2011
(73) Proprietor: Amarantus Therapeutics, Inc., Sunnyvale, CA 94085 (US)
(72) Inventor: COMMISSIONG, John, Sunnyvale CA 94085 (US)
(74) Representative: J A Kemp
(86) International application number: PCT/US2009/038306
(87) International publication number: WO 2009/120810

(56) References cited:
- WO-A2-02/074956
- US-A1- 2002 182 198
- US-A1- 2006 084 619
- LINDHOLM PÄIVI ET AL: "Novel neurotrophic factor CDNF protects and rescues midbrain dopamine neurons in vivo", NATURE, NATURE PUBLISHING GROUP, LONDON, GB, vol. 448, no. 7149, 5 July 2007 (2007-07-05), pages 73-77, XP002507601, ISSN: 0028-0836, DOI: DOI:10.1038/NATURE05957
- PETROVA, P. S. ET AL.: 'MANF: a new mesencephalic, astrocyte-derived neurotrophic factor with selectivity for dopaminergic neurons' J. MOL. NEUROSCI. vol. 20, no. 2, 2003, pages 173 - 188
- DAUER, W. ET AL.: 'Neurotrophic factors and Parkinson's disease: the emergence of a new player?' SCI. STKE. vol. 2007, no. IS.411, 2007, page E60
- ZHOU, C. ET AL.: 'Mesencephalic astrocyte-derived neurotrophic factor enhances nigral gamma- aminobutyric acid release' NEUROREPORT. vol. 17, no. 3, 2006, pages 293 - 297, XP008104489
- PETROVA, P. S. ET AL.: 'Discovering novel phenotype-selective neurotrophic factors to treat neurodegenerative disease' PROG. BRAIN RES. vol. 146, 2004, pages 168 - 183

## Description

### BACKGROUND OF THE INVENTION

The invention relates to compositions and methods for increasing the survival of neurons. The growth, survival, and differentiation of neurons in the peripheral and central nervous systems (PNS and CNS, respectively) are dependent, in part, on target-derived, paracrine, and autocrine neurotrophic factors. Conversely, the lack of neurotrophic factors is thought to play a role in the etiology of neurodegenerative diseases such as Parkinson's disease, Alzheimer's disease, and amyotrophic lateral sclerosis (ALS or Lou Gehrig's disease). In neuronal cell cultures, neurotrophic support is provided by co-culturing with astrocytes or by providing conditioned medium (CM) prepared from astrocytes. Astrocytes of ventral mesencephalic origin exert much greater efficacy in promoting the survival of ventral, mesencephalic dopaminergic neurons, compared with astrocytes from other regions of the CNS, such as the neostriatum and cerebral cortex. In chronic, mesencephalic cultures of 21 days in vitro (DIV) or longer, the percentage of dopaminergic neurons increases from 20% to 60%, coincident with proliferation of a monolayer of astrocytes. In contrast, in conditions in which the proliferation of astrocytes was inhibited, dopaminergic, but not GABAergic neurons, were almost eliminated from the cultures by 5 DIV. These results demonstrate the importance of homotypically-derived astrocytes for the survival and development of adjacent dopaminergic neurons, and suggest that mesencephalic astrocytes are a likely source of a physiological, paracrine neurotrophic factor for mesencephalic dopaminergic neurons.

The repeated demonstration that astrocytes secrete molecules that promote neuronal survival has made astrocytes a focus in the search for therapeutics to treat neurodegenerative diseases. Many laboratories have attempted to isolate astrocyte-derived neurotrophic factors, but have been hindered by a major technical problem: serum is an essential component of the medium for the optimal growth of primary astrocytes in culture, yet the presence of serum interferes with the subsequent purification of factors secreted into the conditioned medium.

Thus, there remains a need for compositions and methods directed to treatment of neuropathic disease.

### SUMMARY OF THE INVENTION

In various aspects of the disclosure compositions and methods are provided related to treating a neurological disorder.

Thus, in accordance with the present invention, there is provided a composition comprising mensencephalic astrocyte-derived neurotrophic factor (MANF), for use in treating Parkinson's Disease, wherein MANF is administered to the substantia nigra region of the brain in a subject.

The present invention also provides for a composition comprising MANF for use in treating Parkinson's Disease, wherein MANF is administered to the ventral mesencephalon region in a subject.

The present invention further provides for a composition for use according to the above, wherein a neural stem cell is transplanted to said region.

In some embodiments of the disclosure, MANF and/or biologically functional fragments thereof are administered to a subject to treat a neurological disorder. In one embodiment, the neurological disorder is Parkinson's Disease.

In another aspect of the disclosure, MANF and/or biologically functional fragments thereof are co-administered to a subject with one or more therapeutic agent. In some embodiments, the one or more therapeutic agent is a neurotrophic factor.

In one embodiment, a method of treating a neurological disorder is provided for increasing the release of GABA from GABAergic terminals in the substantia niagra comprising administering to a subject suffering from Parkinson an effective amount of MANF. In another embodiment, a method of treating method of treating a neurological disorder comprising administering to a subject suffering from the neurological disorder an effective amount of MANF resulting in antagonizing the excitotoxic action of glutamate on DA neurons. In a further embodiment, the neurological disorder is Parkinson's Disease.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**Figure** 1. illustrates cumulative ipsilateral turning behavior of rats after aphetamine dosing; A = VEH + VEH; B= VEH+6OHDA; C=GDNF+6OHDA; D=MANF+6OHDA; E=GDNF+VEH; F=MANF+VEH.
**Figure 2****.** illustrates RT-PCR analysis of MANF1 expression in various tissues, including brain tissues of adult and developing mouse brain.

### DETAILED DESCRIPTION OF THE INVENTION

The disclosure relates generally to compositions and methods for increasing survival of neurons using mesencephalic astrocyte-derived *neurotrophic* factor (MANF). MANF is a new neurotrophic factor that selectively protects DA neurons in vitro and corrects the neurological deficits caused by the degeneration of dopaminergic neurons (DA) neurons on one side of the brain in vivo. The actions of MANF indicate that it can be used to treat Parkinson's Disease (PD). Additionally, MANF is expressed at a high level in the ventral mesencephalon, the same region of the brain where the DA neurons that die in PD are located. MANF also increased the release of GABA from GABAergic terminals in the substantia nigra. GABA antagonizes the excitotoxic action of glutamate on DA neurons. The disclosures herein demonstrate that there are two targets for MANF in the brain of PD patients: 1) The cell body of the DA neuron; 2) The presynaptic GABAergic terminals in the substantia nigra.

In one aspect, the disclosure features a method of treating a patient having a disease or disorder of the nervous system. The method including the step of administering to the patient a dopaminergic neuronal survival-promoting amount of a substantially purified MANF polypeptide.

In a second aspect, the disclosure features a method for preventing dopaminergic neuronal cell death in a mammal. This method includes the step of administering to the mammal a dopaminergic neuronal survival-promoting amount of a substantially purified MANF polypeptide.

In a third aspect method of transplanting cells into the nervous system of a mammal such as a human, including (i) transplanting cells into the nervous system of the mammal; and (ii) administering a dopaminergic neuronal survival-promoting amount of a MANF polypeptide to the mammal in a time window from two to four hours before transplanting the cells to two to four hours after transplanting the cells.

In fourth aspect, the disclosure features another method of transplanting cells into the nervous system of a mammal such as a human. This method includes the steps of: (a) contacting the cells with a MANF polypeptide; and (b) transplanting the cells of step (a) into the nervous system of the mammal. In one embodiment, it is desirable that step (a) and step (b) be performed within four hours of each other.

As demonstrated herein, maintenance, survival or growth of dopaminergic neurons are enhanced through administration of a MANF polypeptide. Dopaminergic neurons of the mesencephalon die in patients having Parkinson's disease. The invention thus provides a treatment of Parkinson's disease. In addition, the use of a MANF polypeptide in the treatment of disorders or diseases of the nervous system in which the loss of dopaminergic neurons is present or anticipated is included in the disclosure.

The discovery that MANF is involved in dopaminergic neuronal survival allows MANF to be used in a variety of diagnostic tests and assays for identification of dopaminergic neuronal survival-promoting drugs. MANF expression can also serve as a diagnostic tool for determining whether a person is at risk for a neurodegenerative disorder. This diagnostic process can lead to the tailoring of drug treatments according to patient genotype (referred to as pharmacogenomics), including prediction of the patient's response (e.g., increased or decreased efficacy or undesired side effects upon administration of a compound or drug).

A fifth aspect of the disclosure features a method for enhancing dopaminergic neuronal survival by administering MANF in combination of one or more additional therapeutic agent. In some embodiments, such therapeutic agent is CDNF, GDNF, MANF2 or a combination thereof. In one embodiment, MANF is administered to the substantia nigra region of the brain in a human. In a further embodiment, MANF is administered to the substantia nigra region of the brain before, during or after transplantation of cells to the brain. In additional embodiments, MANF is administered in combination with one or more additional therapeutic agents as described herein. The substantia nigra is a heterogeneous portion of the midbrain, separating the pes (foot) from the tegmentum (covering), and a major element of the basal ganglia system. In consists of two strongly contrasted ensembles, the pars compacta and adjacent dopaminergic groups, and another ensemble made of the pars reticulata and the pars lateralis. The latter two, along with the pallidal nuclei, are elements of the core of the basal ganglia. The substantia nigra compacta and surrounding tissue is responsible for dopamine production in the brain.

In yet another aspect, the disclosure features a method for determining whether a candidate compound modulates MANF-mediated dopaminergic neuronal survival-promoting activity, including: (a) providing a MANF polypeptide; (b) contacting the MANF polypeptide with the candidate compound; and (c) measuring MANF biological activity, wherein altered MANF biological activity, relative to that of a MANF polypeptide not contacted with the compound, indicates that the candidate compound modulates MANF biological activity. The MANF polypeptide can be in a cell or in a cell-free assay system.

In another aspect, the disclosure features a method for determining whether candidate compound is useful for decreasing neurodegeneration, the method including the steps of: (a) providing a MANF polypeptide; (b) contacting the polypeptide with the candidate compound; and (c) measuring binding of the MANF polypeptide, wherein binding of the MANF polypeptide indicates that the candidate compound is useful for decreasing neurodegeneration.

The disclosure also features screening methods for identifying factors that potentiate or mimic MANF biological activity. In these screening methods for potentiators, the ability of candidate compounds to increase MANF expression, stability, or biological activity is tested using standard techniques. A candidate compound that binds to MANF may act as a potentiating agent. A mimetic (e.g., a compound that binds a MANF receptor) is a compound capable of acting in the absence of a MANF polypeptide.

By "substantially purified" is meant that a polypeptide (e.g., a MANF polypeptide) has been separated from the components that naturally accompany it. Typically, the polypeptide is substantially purified when it is at least 60%, by weight, free from the proteins and naturally-occurring organic molecules with which it is naturally associated. Preferably, the polypeptide is at least 75%, more preferably at least 90%, and most preferably at least 99%, by weight, pure. A substantially purified polypeptide may be obtained, for example, by extraction from a natural source (e.g., a neural cell), by expression of a recombinant nucleic acid encoding the polypeptide, or by chemically synthesizing the protein. Purity can be measured by any appropriate method, e.g., by column chromatography, polyacrylamide gel electrophoresis, or HPLC analysis.

A polypeptide is substantially free of naturally associated components when it is separated from those contaminants that accompany it in its natural state. Thus, a polypeptide which is chemically synthesized or produced in a cellular system different from the cell from which it naturally originates will be substantially free from its naturally associated components. Accordingly, substantially purified polypeptides include those which naturally occur in eukaryotic organisms but are synthesized in E. coli or other prokaryotes.

By "polypeptide" or "protein" is meant any chain of more than two amino acids, regardless of post-translational modification such as glycosylation or phosphorylation.

By "pharmaceutically acceptable excipient" is meant an excipient, carrier, or diluent that is physiologically acceptable to the treated mammal while retaining the therapeutic properties of the polypeptide with which it is administered. One exemplary pharmaceutically acceptable carrier is physiological saline solution. Other physiologically acceptable carriers and their formulations are known to one skilled in the art and described, for example, in Remington: The Science and Practice of Pharmacy, (20th ed.) ed. A. R. Gennaro A R., 2000, Lippencott Williams & Wilkins.

By a compound having "dopaminergic neuronal survival-promoting activity" is the presence of the compound increases survival of dopaminergic neurons by at least two-fold in a dopaminergic neuronal survival assay (such as the one described herein) relative to survival of dopaminergic neurons in the absence of the compound. The increase in the survival of dopaminergic neurons can be by at least three-fold, more preferably by at least four-fold, and most preferably by at least five-fold. The assay can be an in vitro assay or an in vivo assay.

As shown previously (Commissiong et al., US Application Publication No. 2002/0182198, or Serial No. 10/102265), a cell line of mesencephalic origin (termed "VMCL-1 ") secretes a factor that, in turn, promotes differentiation and survival of dopaminergic neurons. This cell line grows robustly in a serum-free medium. Moreover, the CM prepared from these cells contains one or more dopaminergic neuronal survival factors that increase the survival of mesencephalic dopaminergic neurons at least 3-fold, and promotes their development as well.

Commissiong et al. also demonstrated purification of MANF from the VMCL-1 cell line. The protein was isolated as follows. A 3 L volume of VMCL-1 conditioned medium was prepared, and subjected to five sequential steps of column chromatography. At each purification step, each column fraction was tested for biological activity in the bioassay referred to above. An estimate of the effect of each fraction on dopaminergic neuronal survival was done at 24 hour intervals, over a period of five days, and rated on a scale of 1-10. After the fifth purification step, the biologically active fraction and an adjacent inactive fraction were analyzed by SDS-PAGE. The results of the SDS-PAGE analysis revealed a distinctive protein band in the 20 kDa range in the lane from the active fraction. The "active" band was excised and subjected to tryptic digest, and the molecular mass and sequence of each peptide above background were determined by mass spectrometry analysis. The following two peptide sequences were identified: DVTFSPATIE and QIDLSTVDL. A search of the database identified a match for human arginine-rich protein and its mouse orthologue. The predicted protein encoded by the mouse EST sequence is about 95% identical to the predicted human protein. A search of the rat EST database revealed two sequences, one (dbEST Id: 4408547; EST name: EST348489) having significant homology at the amino acid level to the human and mouse proteins. The full-length rat sequence was not in the GenBank database.

Furthermore, Commissiong et al. discovered that human ARP is cleaved such that the arginine-rich amino-terminus is separated from the carboxy-terminus to produce human pro-MANF. The cleaved carboxy-terminal fragment contains a signal peptide, resulting in the secretion of human MANF from the cell.

### Protein Therapy

In one aspect of the disclosure MANF or biologically functional fragments thereof are administered to a subject to treat a subject prophylactically or a subject suffering from a neuropathy. For example, in some embodiments, a subject is treated to enhance maintenance and/or promote growth of dopaminergic neurons. Various dosage formulations contemplated for use in methods of the invention are disclosed herein *infra.*

In various embodiments, a therapeutic approach within the disclosure involves administration of a recombinant MANF polypeptide, either directly to the site of a potential or actual cell loss (for example, by injection) or systemically (for example, by any conventional recombinant protein administration technique).

In one embodiment, administration is to the substantia nigra region of the brain and/or the ventral mesencephalon region.

An additional embodiment of the disclosure relates to the administration of a pharmaceutical composition, in conjunction with a pharmaceutically acceptable carrier, for any of the therapeutic effects discussed above. Such pharmaceutical compositions may consist of MANF polypeptides, antibodies to MANF polypeptides, and/or mimetics and agonists of MANF polypeptides.

In various embodiments of the disclosure, both the secreted and/or unsecreted forms of MANF are administered to a subject to enhance dopaminergic neuronal growth or survival. The secreted form and the unsecreted form of MANF (collectively referred to as MANF polypeptides) have neurotrophic activity and are useful as neurotrophic factor for the treatment of a neurodegenerative disease such as Parkinson's Disease and for improving dopaminergic neuronal survival during or following transplantation into a human. MANF polypeptides can also be used to improve the in vitro production of neurons for transplantation. In another use, MANF polypeptides can be used for the identification of compounds that modulate or mimic MANF's dopaminergic neuronal survival-promoting activity. MANF polypeptides can also be used to identify MANF receptors. Each of these uses is described in greater detail below.

The discovery of MANF as a neurotrophic factor that promotes the survival of dopaminergic neurons allows for its use for the therapeutic treatment of neurodegenerative diseases such as Parkinson's disease.

In one embodiment, a method is provided of treating Parkinson by increasing the release of GABA from GABAergic terminals in the substantia niagra, the method comprising administering to a subject suffering from Parkinson an effective amount of MANF. In a further embodiment, administration comprises co-administering one or more additional therepeutic agents described herein. In yet a further embodiment, a method is provided of treating Parkinson by antagonizing the excitotoxic action of glutamate on DA neurons comprising administering to a subject suffering from Parkinson an effective amount of MANF and alternatively, MANF and one or more additional therapeutic agents disclosed herein.

In another embodiment, a MANF polypeptide is administered to a subject at the site that cells are transplanted. The administration of the MANF polypeptide can be performed before, during, or after the transplantation of the cells. Preferably, the two steps are within about four hours of each other. If desirable, the MANF polypeptide can be repeatedly administered to the subject at various intervals before and/or after cell transplantation. This protective administration of the MANF polypeptide may occur months or even years after the cell transplantation. Transplanting methods and compositions useful in various aspects of the invention include those disclosed in U.S. Patent Application Publication NOs: 20080058221; 20080050728; 20080014246; 20070275938).

For example, administration of MANF to an animal model of Parkinson's Disease (brain lesion-induced rodent) resulted in a strikingly therapeutic effect. The dopaminergic neurons of the brain were lesioned by a neurotoxin, 6-hydroxydopamine (6-OHDA), that was injected close to dopaminergic neurons in the substantia nigra, and selectively taken up into dopaminergic neurons via the dopamine transporter (DAT). 6-OHDA selectively destroys dopaminergic neurons on the injected side of the brain. An imbalance created in the release of DA causes the animal to rotate to the intact side of the brain (Mendez and Finn, 1975). Table 1 shows the difference in number of turns between vehicle (VEH) and treatment with GDNF or MANF:

**TABLE 1:**

| Group | Number of Turns in 120 minutes |
|---|---|
| VEH + VEH | 20 ± 45 |
| VEH + 6-OHDA | 950 ± 150 |
| GDNF + 6-OHDA | 50 ± 75 |
| MANF + 6-OHDA | 150 ± 55 |

The results demonstrate that administration of MANF reduces cumulative turning during 120 minutes of monitoring. The more turns, the more severe the effects of the brain-induced lesions in the striatum.

In addition to its administration to a human or other mammal, a MANF polypeptide can also be used in culture to improve the survival of neurons during their production any time prior to transplantation. In one example, the cells to be transplanted are suspended in a pharmaceutical carrier that also includes a survival-promoting amount of a MANF polypeptide. A MANF polypeptide can also be administered to the cultures earlier in the process (e.g., as the neurons are first differentiating). It is understood that the neurons need not be primary dopaminergic neurons. Neurons (e.g., dopaminergic neurons) that are differentiated, either in vitro or in vivo, from stem cells or any other cell capable of producing neurons can be cultured in the presence of a MANF polypeptide during their production and maintenance..

To add a MANF polypeptide to cells in order to prevent neuronal death, it is preferable to obtain sufficient amounts of a recombinant MANF polypeptide from cultured cell systems that can express the protein. A preferred MANF polypeptide is human MANF, but MANF polypeptides derived from other animals (e.g., pig, rat, mouse, dog, baboon, cow, and the like) can also be used. Delivery of the protein to the affected tissue can then be accomplished using appropriate packaging or administrating systems. Alternatively, small molecule analogs may be used and administered to act as MANF agonists and in this manner produce a desired physiological effect.

### Gene Therapy

Broadly, gene therapy seeks to transfer new genetic material to the cells of a patient with resulting therapeutic benefit to the patient. Such benefits include treatment or prophylaxis of a broad range of diseases, disorders and other conditions.

The disclosure provides a method to deliver a transgene to the CNS in a subject by administering a recombinant neurotropic viral vector containing a transgene, wherein the delivery is under conditions that favor expression of the transgene at a site distal to the site of administration. The delivery may also result in expression of the transgene at the site of administration. For example, methods of gene therapy known in the art are disclosed in US Patent Application No. 20090069261, which is hereby incorporated by reference in its entirety.

Unless specifically indicated to the contrary, expression of the transgene is not limited to translation to a polypeptide or protein but also includes replication and/or transcription of the transgene polynucleotide.

In another aspect, the invention provides a method of delivering a therapeutic transgene product to a target cell of the CNS, which is a neuron or a glial cell, in a mammal afflicted with a disorder, such as Parkinson's Disease.

Ex vivo gene therapy approaches involve modification of isolated cells, which are then infused, grafted or otherwise transplanted into the patient. See, e.g., U.S. Pat. Nos. 4,868,116, 5,399,346 and 5,460,959. In vivo gene therapy seeks to directly target host patient tissue in vivo.

Viruses useful as gene transfer vectors include papovavirus, adenovirus, vaccinia virus, adeno-associated virus, herpesvirus, and retroviruses. Suitable retroviruses include the group consisting of HIV, SIV, FIV, EIAV, MoMLV.

Preferred viruses for treatment of disorders of the central nervous system are lentiviruses and adeno-associated viruses. Both types of viruses can integrate into the genome without cell divisions, and both types have been tested in pre-clinical animal studies for indications in the nervous system, in particular in the central nervous system.

Methods for preparation of AAV are described in the art, e.g. U.S. Pat. No. 5,677,158, U.S. Pat. No. 6,309,634, and U.S. Pat. No. 6,451,306 describe examples of delivery of MV to the central nervous system.

In the methods of the disclosure, AAV of any serotype can be used. In certain embodiments, AAV of any serotype can be used so long as the vector is capable of undergoing retrograde axonal transport in a disease-compromised brain, or axonal transport in a non-compromised brain. The serotype of the viral vector used in certain embodiments of the invention is selected from the group consisting from AAV1, AAV2, AAV3, AAV4, MV5, AAV6, AAV7, and AAV8 (see, e.g., Gao et al. (2002) PNAS, 99:11854-11859; and Viral Vectors for Gene Therapy: Methods and Protocols, ed. Machida, Humana Press, 2003). Other serotype besides those listed herein can be used. Furthermore, pseudotyped AAV vectors may also be utilized in the methods described herein. Pseudotyped AAV vectors are those which contain the genome of one AAV serotype in the capsid of a second AAV serotype; for example, an AAV vector that contains the AAV2 capsid and the AAV1 genome or an AAV vector that contains the AAV5 capsid and the AAV 2 genome (Auricchio et al., (2001) Hum. Mol. Genet., 10(26):3075-81).

AAV vectors are derived from single-stranded (ss) DNA parvoviruses that are nonpathogenic for mammals (reviewed in Muzyscka (1992) Curr. Top. Microb. Immunol., 158:97-129). Briefly, AAV-based vectors have the rep and cap viral genes that account for 96% of the viral genome removed, leaving the two flanking 145-basepair (bp) inverted terminal repeats (ITRs), which are used to initiate viral DNA replication, packaging and integration. In the absence of helper virus, wild-type AAV integrates into the human host-cell genome with preferential site-specificity at chromosome 19q13.3 or it may remain expressed episomally. A single AAV particle can accommodate up to 5 kb of ssDNA, therefore leaving about 4.5 kb for a transgene and regulatory elements, which is typically sufficient. However, trans-splicing systems as described, for example, in U.S. Pat. No. 6,544,785, may nearly double this limit.

A special and preferred type of retroviruses include the lentiviruses which can transduce a cell and integrate into its genome without cell division. Thus preferably the vector is a replication-defective lentivirus particle. Such a lentivirus particle can be produced from a lentiviral vector comprising a 5' lentiviral LTR, a tRNA binding site, a packaging signal, a promoter operably linked to a polynucleotide signal encoding said fusion protein, an origin of second strand DNA synthesis and a 3' lentiviral LTR. Methods for preparation and in vivo administration of lentivirus to neural cells are described in US 20020037281 (Methods for transducing neural cells using lentiviral vectors) and US 20020187951 (Lentiviral-mediated growth factor gene therapy for neurodegenerative diseases).

Construction of vectors for recombinant expression of MANF for use in the invention may be accomplished using conventional techniques which do not require detailed explanation to one of ordinary skill in the art. For review, however, those of ordinary skill may wish to consult Maniatis et al., in Molecular Cloning: A Laboratory Manual, Cold Spring Harbor Laboratory, (NY 1982).

Chimeric expression constructs used in the present invention may be created, e.g. by amplifying the desired fragments (a signal sequence and a MANF coding sequence) by PCR and fusing these in overlapping PCR. As several of the preferred signal sequences are relatively short, the 5' PCR primer used for amplifying the MANF coding sequence may include the sequence coding for the signal sequence as well as a TATA box and other regulatory elements.

Briefly, construction of recombinant expression vectors employs standard ligation techniques. For analysis to confirm correct sequences in vectors constructed, the genes are sequences using, for example, the method of Messing, et al., (Nucleic Acids Res., 9: 309-, 1981), the method of Maxam, et al., (Methods in Enzymology, 65: 499, 1980), or other suitable methods which will be known to those skilled in the art.

Expression of a gene is controlled at the transcription, translation or post-translation levels. Transcription initiation is an early and critical event in gene expression. This depends on the promoter and enhancer sequences and is influenced by specific cellular factors that interact with these sequences. The transcriptional unit of many genes consists of the promoter and in some cases enhancer or regulator elements (Banerji et al., Cell 27: 299 (1981); Corden et al., Science 209: 1406 (1980); and Breathnach and Chambon, Ann. Rev. Biochem. 50: 349 (1981)). For retroviruses, control elements involved in the replication of the retroviral genome reside in the long terminal repeat (LTR) (Weiss et al., eds., The molecular biology of tumor viruses: RNA tumor viruses, Cold Spring Harbor Laboratory, (NY 1982)). Moloney murine leukemia virus (MLV) and Rous sarcoma virus (RSV) LTRs contain promoter and enhancer sequences (Jolly et al., Nucleic Acids Res. 11: 1855 (1983); Capecchi et al., In: Enhancer and eukaryotic gene expression, Gulzman and Shenk, eds., pp. 101-102, Cold Spring Harbor Laboratories (NY 1991). Promoters that show long-term activity and are tissue- and even cell-specific are used in some embodiments. Nonlimiting examples of promoters include, but are not limited to, the cytomegalovirus (CMV) promoter (Kaplitt et al. (1994) Nat. Genet. 8:148-154), CMV/human.beta.3-globin promoter (Mandel et al. (1998) J. Neurosci. 18:4271-4284), GFAP promoter (Xu et al. (2001) Gene Ther. 8:1323-1332), the 1.8-kb neuron-specific enolase (NSE) promoter (Klein et al. (1998) Exp. Neurol. 150:183-194), chicken beta actin (CBA) promoter (Miyazaki (1989) Gene 79:269-277), the .beta.-glucuronidase (GUSB) promoter (Shipley et al. (1991) Genetics 10:1009-1018), and ubiquitin promoters such as those isolated from human ubiquitin A, human ubiquitin B, and human ubiquitin C as described in U.S. Pat. No. 6,667,174. To prolong expression, other regulatory elements may additionally be operably linked to the transgene, such as, e.g., the Woodchuck Hepatitis Virus Post-Regulatory Element (WPRE) (Donello et al. (1998) J. Virol. 72:5085-5092) or the bovine growth hormone (BGH) polyadenylation site.

Promoter and enhancer regions of a number of non-viral promoters have also been described (Schmidt et al., Nature 314: 285 (1985); Rossi and decrombrugghe, Proc. Natl. Acad. Sci. USA 84: 5590-5594 (1987)). Methods for maintaining and increasing expression of transgenes in quiescent cells include the use of promoters including collagen type I (1 and 2) (Prockop and Kivirikko, N. Eng. J. Med. 311: 376 (1984); Smith and Niles, Biochem. 19: 1820 (1980); de Wet et al., J. Biol. Chem., 258: 14385 (1983)), SV40 and LTR promoters.

According to one embodiment of the disclosure, the promoter is a constitutive promoter selected from the group consisting of: ubiquitin promoter, CMV promoter, JeT promoter (U.S. Pat. No. 6,555,674), SV40 promoter, and Elongation Factor 1 alpha promoter (EF1-alpha). Examples of inducible/repressible promoters include: Tet-On, Tet-Off, Rapamycin-inducible promoter, Mxl.

In addition to using viral and non-viral promoters to drive transgene expression, an enhancer sequence may be used to increase the level of transgene expression. Enhancers can increase the transcriptional activity not only of their native gene but also of some foreign genes (Armelor, Proc. Natl. Acad. Sci. USA 70: 2702 (1973)). For example, in the present invention collagen enhancer sequences may be used with the collagen promoter 2 (I) to increase transgene expression. In addition, the enhancer element found in SV40 viruses may be used to increase transgene expression. This enhancer sequence consists of a 72 base pair repeat as described by Gruss et al., Proc. Natl. Acad. Sci. USA 78: 943 (1981); Benoist and Chambon, Nature 290: 304 (1981), and Fromm and Berg, J. Mol. Appl. Genetics, 1: 457 (1982). This repeat sequence can increase the transcription of many different viral and cellular genes when it is present in series with various promoters (Moreau et al., Nucleic Acids Res. 9: 6047 (1981).

Further expression enhancing sequences include but are not limited to Woodchuck hepatitis virus post-transcriptional regulation element, WPRE, SP163, rat Insulinil-intron or other introns, CMV enhancer, and Chicken [beta]-globin insulator or other insulators.

Transgene expression may also be increased for long term stable expression using cytokines to modulate promoter activity. Several cytokines have been reported to modulate the expression of transgene from collagen 2 (I) and LTR promoters (Chua et al., connective Tissue Res., 25: 161-170 (1990); Elias et al., Annals N.Y. Acad. Sci., 580: 233-244 (1990)); Seliger et al., J. Immunol. 141: 2138-2144 (1988) and Seliger et al., J. Virology 62: 619-621 (1988)). For example, transforming growth factor (TGF), interleukin (IL)-I, and interferon (INF) down regulate the expression of transgenes driven by various promoters such as LTR. Tumor necrosis factor (TNF) and TGF 1 up regulate, and may be used to control, expression of transgenes driven by a promoter. Other cytokines that may prove useful include basic fibroblast growth factor (bFGF) and epidermal growth factor (EGF).

Collagen promoter with the collagen enhancer sequence (Coll (E)) may also be used to increase transgene expression by suppressing further any immune response to the vector which may be generated in a treated brain notwithstanding its immune-protected status. In addition, anti-inflammatory agents including steroids, for example dexamethasone, may be administered to the treated host immediately after vector composition delivery and continued, preferably, until any cytokine-mediated inflammatory response subsides. An immunosuppression agent such as cyclosporin may also be administered to reduce the production of interferons, which downregulates LTR promoter and Coll (E) promoter-enhancer, and reduces transgene expression.

The vector may comprise further sequences such as a sequence coding for the Cre-recombinase protein, and LoxP sequences. A further way of ensuring temporary expression of the neublastin is through the use of the Cre-LoxP system which results in the excision of part of the inserted DNA sequence either upon administration of Cre-recombinase to the cells (Daewoong et al, Nature Biotechnology 19:929-933) or by incorporating a gene coding for the recombinase into the virus construct (Pluck, Int J Exp Path, 77:269-278). Incorporating a gene for the recombinase in the virus construct together with the LoxP sites and a structural gene (a neublastin in the present case) often results in expression of the structural gene for a period of approximately five days.

In an illustrative embodiment, AAV is AAV2 or AAV1. Adeno-associated virus of many serotypes, especially AAV2, have been extensively studied and characterized as gene therapy vectors. Those skilled in the art will be familiar with the preparation of functional AAV-based gene therapy vectors. Numerous references to various methods of AAV production, purification and preparation for administration to human subjects can be found in the extensive body of published literature (see, e.g., Viral Vectors for Gene Therapy: Methods and Protocols, ed. Machida, Humana Press, 2003). Additionally, AAV-based gene therapy targeted to cells of the CNS has been described in U.S. Pat. Nos. 6,180,613 and 6,503,888. Additional exemplary AAV vectors are recombinant AAV2/1, AAV2/2, AAV2/5, AAV2/7 and AAV2/8 serotype vectors encoding human protein.

For some CNS gene therapy applications, it may be necessary to control transcriptional activity. To this end, pharmacological regulation of gene expression with viral vectors can been obtained by including various regulatory elements and drug-responsive promoters as described, for example, in Haberma et al. (1998) Gene Ther. 5:1604-16011; and Ye et al. (1995) Science 283:88-91.

In the methods of this invention, the viral vector can be administered by contacting an terminal axonal ending of a neuron with a composition containing a viral vector carrying the transgene, allowing the viral particle to be endocytosed and transported intracellularly (retrogradely) along the axon to the cell body of the neuron; allowing the therapeutic transgene product to be expressed, wherein the therapeutic transgene product thereby alleviates pathology in the subject. In certain embodiments, the concentration of the vector in the composition is at least: (a) 5, 6, 7, 8, 8.4, 9, 9.3, 10, 15, 20, 25, or 50 (10¹² gp/ml); (b) 5, 6, 7, 8, 8.4, 9, 9.3, 10, 15, 20, 25, or 50 (X10⁹ tu/ml); or (c) 5, 6, 7, 8, 8.4, 9, 9.3, 10, 15, 20, 25, or 50 (X10¹⁰ iu/ml).

In additional methods of this invention, the viral vector can be administered by contacting the cell body of a neuron with a composition containing a viral vector carrying the transgene, allowing the viral particle to be endocytosed, allowing the therapeutic transgene product to be expressed and transported anterogradely intracellularly along the axon to the axon terminal of the neuron, wherein the therapeutic transgene product thereby alleviates pathology in the subject. In certain embodiments, the concentration of the vector in the composition is at least: (a) 5, 6, 7, 8, 8.4, 9, 9.3, 10, 15, 20, 25, or 50 (10¹² gp/ml); (b) 5, 6, 7, 8, 8.4, 9, 9.3, 10, 15, 20, 25, or 50 (X10⁹ tu/ml); or (c) 5, 6, 7, 8, 8.4, 9, 9.3, 10, 15, 20, 25, or 50 (10¹⁰ iu/ml).

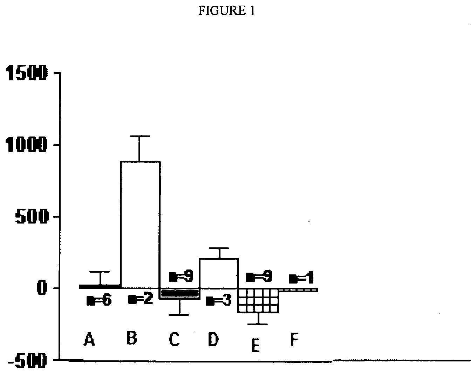

For identification of structures in the human brain, see, e.g., The Human Brain: Surface, Three-Dimensional Sectional Anatomy With MRI, and Blood Supply, 2nd ed., eds. Deuteron et al., Springer Vela, 1999; Atlas of the Human Brain, eds. Mai et al., Academic Press; 1997; and Co-Planar Sterotaxic Atlas of the Human Brain: 3-Dimensional Proportional System: An Approach to Cerebral Imaging, eds. Tamarack et al., Thyme Medical Pub., 1988. For identification of structures in the mouse brain, see, e.g., The Mouse Brain in Sterotaxic Coordinates, 2nd ed., Academic Press, 2000. FIG. 1 schematically shows the spinal cord and its four subdivisions: cervical, thoracic, lumbar and sacral.

A further important parameter is the dosage of MANF to be delivered into the target tissue. In this regard, "unit dosage" refers generally to the concentration of Neurturin/ml of MANF composition. For viral vectors, the MANF concentration may be defined by the number of viral particles/ml of neurotrophic composition. Optimally, for delivery of MANF using a viral expression vector, each unit dosage of MANF will comprise 2.5 to 25 .mu.L of a MANF composition, wherein the composition includes a viral expression vector in a pharmaceutically acceptable fluid and provides from 10¹⁰ up to 10.sup.15 MANF expressing viral particles per ml of MANF composition. Such high titers are particularly useful for adeno-associated virus. For lentivirus, the titer is normally lower, such as from 10.sup.8 to 10.sup.10 transducing units per ml (TU/mL) determined as described in the examples.

In a preferred embodiment, the administration site is the striatum of the brain, in particular the caudate and/or the putamen. Injection into the putamen can label target sites located in various distant regions of the brain, for example, the globus pallidus, amygdala, subthalamic nucleus or the substantia nigra. Transduction of cells in the pallidus commonly causes retrograde labelling of cells in the thalamus. In a preferred embodiment the (or one of the) target site(s) is the substantia nigra. Injection may also be into both the striatum and the substantia nigra.

Within a given target site, the vector system may transduce a target cell. The target cell may be a cell found in nervous tissue, such as a neuron, astrocyte, oligodendrocyte, microglia or ependymal cell. In a preferred embodiment, the target cell is a neuron, in particular a TH positive neuron.

The vector system is preferably administered by direct injection. Methods for injection into the brain (in particular the striatum) are well known in the art (Bilang-Bleuel et al (1997) Proc. Acad. Natl. Sci. USA 94:8818-8823; Choi-Lundberg et al (1998) Exp. Neurol. 154:261-275; Choi-Lundberg et al (1997) Science 275:838-841; and Mandel et al (1997)) Proc. Acad. Natl. Sci. USA 94:14083-14088). Stereotaxic injections may be given.

As mentioned above, for transduction in tissues such as the brain, it is necessary to use very small volumes, so the viral preparation is concentrated by ultracentrifugation. The resulting preparation should have at least 10.sup.8 t.u./ml, preferably from 10⁸ to 10¹⁰ t.u./ml, more preferably at least 10⁹ t.u./ml. (The titer is expressed in transducing units per ml (t.u./ml) as described in example 2). It has been found that improved dispersion of transgene expression can be obtained by increasing the number of injection sites and decreasing the rate of injection (Horellou and Mallet (1997) as above). Usually between 1 and 10 injection sites are used, more commonly between 2 and 6. For a dose comprising 1-5X10⁹ t.u./ml, the rate of injection is commonly between 0.1 and 10 .mu.1/min, usually about 1 .mu.1/min.

The MANF composition is delivered to each delivery cell site in the target tissue by microinjection, infusion, scrape loading, electroporation or other means suitable to directly deliver the composition directly into the delivery site tissue through a surgical incision. The delivery is accomplished slowly, such as over a period of about 5-10 minutes (depending on the total volume of MANF composition to be delivered).

### Modifications

In another aspect of the invention, MANF is modified prior to administration to a subject.

Modified proteins of the present invention may possess deletions and/or substitutions of amino acids; thus, a protein with a deletion, a protein with a substitution, and a protein with a deletion and a substitution are modified proteins. In some embodiments these modified proteins may further include insertions or added amino acids, such as with fusion proteins or proteins with linkers, for example.

Substitutional or replacement variants typically contain the exchange of one amino acid for another at one or more sites within the protein and may be designed to modulate one or more properties of the polypeptide, particularly to reduce its immunogenicity/antigenicity, reduce any side effects in a subject, or increase its efficacy. Substitutions of this kind preferably are conservative, that is, one amino acid is replaced with one of similar shape and charge. Conservative substitutions are well known in the art and include, for example, the changes of: alanine to serine; arginine to lysine; asparagine to glutamine or histidine; aspartate to glutamate; cysteine to serine; glutamine to asparagine; glutamate to aspartate; glycine to proline; histidine to asparagine or glutamine; isoleucine to leucine or valine; leucine to valine or isoleucine; lysine to arginine; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine. An antigenic region of a polypeptide may be substituted for a less antigenic region; the less antigenic region may contain residues that are identical to the corresponding residues in the native protein, yet also contain some conservative substitutions and/or nonconservative substitutions.

In addition to a deletion or substitution, a modified protein may possess an insertion of residues, which typically involves the addition of at least one residue in the polypeptide. This may include the insertion of a targeting peptide or polypeptide or simply a single residue. Terminal additions, called fusion proteins, are discussed below.

The term "biologically functional equivalent" is well understood in the art and is further defined in detail herein. Accordingly, sequences that have between about 70% and about 80%, or between about 81% and about 90%, or even between about 91% and about 99% of amino acids that are identical or functionally equivalent to the amino acids of a native polypeptide are included, provided the biological activity of the protein is maintained. A modified protein may be biologically functionally equivalent to its native counterpart. In various embodiments, a biologically functional equivalent of MANF is administered in a therapeutically effective amount.

It also will be understood that amino acid and nucleic acid sequences may include additional residues, such as additional N- or C-terminal amino acids or 5' or 3' sequences, and yet still be essentially as set forth in one of the sequences disclosed herein, so long as the sequence meets the criteria set forth above, including the maintenance of biological protein activity where protein expression is concerned. The addition of terminal sequences particularly applies to nucleic acid sequences that may, for example, include various non-coding sequences flanking either of the 5' or 3' portions of the coding region or may include various internal sequences, i.e., introns, which are known to occur within genes.

The following is a discussion based upon changing of the amino acids of a protein to create an equivalent, or even an improved, second-generation molecule. For example, certain amino acids may be substituted for other amino acids in a protein structure without appreciable loss of interactive binding capacity with structures such as, for example, binding sites to substrate molecules. Since it is the interactive capacity and nature of a protein that defines that protein's biological functional activity, certain amino acid substitutions can be made in a protein sequence, and in its underlying DNA coding sequence, and nevertheless produce a protein with like properties. It is thus contemplated by the inventors that various changes may be made in the DNA sequences of genes without appreciable loss of their biological utility or activity, as discussed below. Table 1 shows the codons that encode particular amino acids. A proteinaceous molecule has "homology" or is considered "homologous" to a second proteinaceous molecule if one of the following "homology criteria" is met: 1) at least 30% of the proteinaceous molecule has sequence identity at the same positions with the second proteinaceous molecule; 2) there is some sequence identity at the same positions with the second proteinaceous molecule and at the nonidentical residues, at least 30% of them are conservative differences, as described herein, with respect to the second proteinaceous molecule; or 3) at least 30% of the proteinaceous molecule has sequence identity with the second proteinaceous molecule, but with possible gaps of nonidentical residues between identical residues. As used herein, the term "homologous" may equally apply to a region of a proteinaceous molecule, instead of the entire molecule. If the term "homology" or "homologous" is qualified by a number, for example, "50% homology" or "50% homologous," then the homology criteria, with respect to 1), 2), and 3), is adjusted from "at least 30%" to "at least 50%." Thus it is contemplated that there may homology of at least 30%, 35%, 40%, 45%, 50%, 55%, 60%, 65%, 70%, 75%, 80%, 85%, 90%, 95%, or more between two proteinaceous molecules or portions of proteinaceous molecules.

In making such changes, the hydropathic index of amino acids may be considered. The importance of the hydropathic amino acid index in conferring interactive biologic function on a protein is generally understood in the art (Kyte & Doolittle, 1982). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like.

It also is understood in the art that the substitution of like amino acids can be made effectively on the basis of hydrophilicity. U.S. Pat. No. 4,554,101, states that the greatest local average hydrophilicity of a protein, as governed by the hydrophilicity of its adjacent amino acids, correlates with a biological property of the protein. As detailed in U.S. Pat. No. 4,554,101, the following hydrophilicity values have been assigned to amino acid residues: arginine (+3.0); lysine (+3.0); aspartate (+3.0.+-.1); glutamate (+3.0.+-.1); serine (+0.3); asparagine (+0.2); glutamine (+0.2); glycine (0); threonine (-0.4); proline (-0.5.+-.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

It is understood that an amino acid can be substituted for another having a similar hydrophilicity value and still produce a biologically equivalent and immunologically equivalent protein. In such changes, the substitution of amino acids whose hydrophilicity values are within .+-.2 is preferred, those that are within .+-.1 are particularly preferred, and those within .+-.0.5 are even more particularly preferred.

As outlined above, amino acid substitutions generally are based on the relative similarity of the amino acid side-chain substituents, for example, their hydrophobicity, hydrophilicity, charge, size, and the like. Exemplary substitutions that take into consideration the various foregoing characteristics are well known to those of skill in the art and include: arginine and lysine; glutamate and aspartate; serine and threonine; glutamine and asparagine; and valine, leucine and isoleucine.

Another embodiment for the preparation of modified polypeptides according to the disclosure is the use of peptide mimetics. Mimetics are peptide-containing molecules that mimic elements of protein secondary structure. See, e.g., Johnson (1993). The underlying rationale behind the use of peptide mimetics is that the peptide backbone of proteins exists chiefly to orient amino acid side chains in such a way as to facilitate molecular interactions, such as those of antibody and antigen. A peptide mimetic is expected to permit molecular interactions similar to the natural molecule. These principles may be used, in conjunction with the principles outline above, to engineer second generation modified protein molecules having many of the natural properties of a native protein, but with altered and, in some cases, even improved characteristics.

### a. Fusion Proteins

A specialized kind of insertional variant is the fusion protein comprising MANF or biologically functional variant thereof. This molecule generally has all or a substantial portion of the native molecule, linked at the N- or C-terminus, to all or a portion of a second polypeptide. For example, fusions typically employ leader sequences from other species to permit the recombinant expression of a protein in a heterologous host. Another useful fusion includes the addition of an immunologically active domain, such as an antibody epitope or other tag, to facilitate targeting or purification of the fusion protein. The use of 6xHis and GST (glutathione S transferase) as tags is well known. Inclusion of a cleavage site at or near the fusion junction will facilitate removal of the extraneous polypeptide after purification. Other useful fusions include linking of functional domains, such as active sites from enzymes such as a hydrolase, glycosylation domains, cellular targeting signals or transmembrane regions.

### b. Conjugated Proteins

In one aspect of the disclosure, MANF or biologically functional fragments thereof are conjugated. For example, in various embodiments, the present invention further provides conjugated polypeptides, such as translated proteins, polypeptides and peptides that are linked to at least one agent to form an conjugate. Of particular use are antibody conjugates, in which the antibody portion targets the agent to a particular site. In order to increase the efficacy of antibody molecules as diagnostic or therapeutic agents, it is conventional to link or covalently bind or complex at least one desired molecule or moiety. Such a molecule or moiety may be, but is not limited to, at least one effector or reporter molecule. Effector molecules comprise molecules having a desired activity, e.g., cell growth activity or cytotoxic activity. Non-limiting examples of effector molecules which have been attached to antibodies include growth agents, toxins, anti-tumor agents, therapeutic enzymes, radio-labeled nucleotides, antiviral agents, chelating agents, cytokines, growth factors, and oligo- or poly-nucleotides. By contrast, a reporter molecule is defined as any moiety that may be detected using an assay. Non-limiting examples of reporter molecules which have been conjugated to antibodies include enzymes, radiolabels, haptens, fluorescent labels, phosphorescent molecules, chemiluminescent molecules, chromophores, luminescent molecules, photoaffinity molecules, colored particles or ligands, such as biotin.

Certain examples of antibody conjugates are those conjugates in which the antibody is linked to a detectable label. "Detectable labels" are compounds and/or elements that can be detected due to their specific functional properties, and/or chemical characteristics, the use of which allows the antibody to which they are attached to be detected, and/or further quantified if desired. Another such example is the formation of a conjugate comprising an antibody linked to a cytotoxic or anti-cellular agent, and may be termed "immunotoxins."

### i. Linkers/Coupling Agents

In another aspect of the disclosure, MANF or one or more biologically functional fragments thereof are joined in a fusion as described above or through a linker or coupling agent as follows. Examples of linker types that can be used to conjugate MANF include, but are not limited to, hydrazones, thioethers, esters, disulfides and peptide-containing linkers. A linker can be chosen that is, for example, susceptible to cleavage by low pH or susceptible to cleavage by proteases, such as cathepsins (e.g., cathepsins B, C, D). For example, multiple peptides or polypeptides may be joined via a biologically-releasable bond, such as a selectively-cleavable linker or amino acid sequence. For example, peptide linkers that include a cleavage site for an enzyme preferentially located or active within a tumor environment are contemplated. Exemplary forms of such peptide linkers are those that are cleaved by urokinase, plasmin, thrombin, Factor IXa, Factor Xa, or a metallaproteinase, such as collagenase, gelatinase, or stromelysin. Alternatively, peptides or polypeptides may be joined to an adjuvant. Amino acids such as selectively-cleavable linkers, synthetic linkers, or other amino acid sequences may be used to separate proteinaceous moieties.

### 4. Protein Purification

While some of the embodiments of the disclosure involve recombinant proteins, the disclosure concerns also methods and processes for purifying proteins, including endogenous polypeptides and peptides and recombinant polypeptides and peptides. Generally, these techniques involve, at one level, the crude fractionation of the cellular milieu to polypeptide and non-polypeptide fractions. Having separated the polypeptide from other proteins, the polypeptide of interest may be further purified using chromatographic and electrophoretic techniques to achieve partial or complete purification (or purification to homogeneity). Analytical methods particularly suited to the preparation of a pure peptide are ion-exchange chromatography, exclusion chromatography; polyacrylamide gel electrophoresis; isoelectric focusing. A particularly efficient method of purifying peptides is fast protein liquid chromatography or even HPLC. In addition, the conditions under which such techniques are executed may be affect characteristics, such as functional activity, of the purified molecules.

Certain aspects of the present disclosure concern the purification, and in particular embodiments, the substantial purification, of an encoded protein or peptide. The term "purified protein or peptide" as used herein, is intended to refer to a composition, isolatable from other components, wherein the protein or peptide is purified to any degree relative to its naturally-obtainable state. A purified protein or peptide therefore also refers to a protein or peptide, free from the environment in which it may naturally occur. A "substantially purified" protein or peptide

Generally, "purified" will refer to a protein or peptide composition that has been subjected to fractionation to remove various other components, and which composition substantially retains its expressed biological activity. Where the term "substantially purified" is used, this designation will refer to a composition in which the protein or peptide forms the major component of the composition, such as constituting about 50%, about 60%, about 70%, about 80%, about 90%, about 95%, about 96%, about 97%, about 98%, about 99%, about 99.2%, about 99.4%, about 99.6%, about 99.8%, about 99.9% or more of the proteins in the composition.

Various methods for quantifying the degree of purification of the protein or peptide will be known to those of skill in the art in light of the present disclosure. These include, for example, determining the specific activity of an active fraction, or assessing the amount of polypeptides within a fraction by SDS/PAGE analysis. A preferred method for assessing the purity of a fraction is to calculate the specific activity of the fraction, to compare it to the specific activity of the initial extract, and to thus calculate the degree of purity, herein assessed by a "-fold purification number." The actual units used to represent the amount of activity will, of course, be dependent upon the particular assay technique chosen to follow the purification and whether or not the expressed protein or peptide exhibits a detectable activity.

Various techniques suitable for use in protein purification will be well known to those of skill in the art. These include, for example, precipitation with ammonium sulphate, PEG, antibodies and the like or by heat denaturation, followed by centrifugation; chromatography steps such as ion exchange, gel filtration, reverse phase, hydroxylapatite and affinity chromatography; isoelectric focusing; gel electrophoresis; and combinations of such and other techniques. As is generally known in the art, it is believed that the order of conducting the various purification steps may be changed, or that certain steps may be omitted, and still result in a suitable method for the preparation of a substantially purified protein or peptide.

There is no general requirement that the protein or peptide always be provided in their most purified state. Indeed, it is contemplated that less substantially purified products will have utility in certain embodiments. Partial purification may be accomplished by using fewer purification steps in combination, or by utilizing different forms of the same general purification scheme. For example, it is appreciated that a cation-exchange column chromatography performed utilizing an HPLC apparatus will generally result in a greater "-fold" purification than the same technique utilizing a low pressure chromatography system. Methods exhibiting a lower degree of relative purification may have advantages in total recovery of protein product, or in maintaining the activity of an expressed protein.

It is known that the migration of a polypeptide can vary, sometimes significantly, with different conditions of SDS/PAGE (Capaldi et al., 1977). It will therefore be appreciated that under differing electrophoresis conditions, the apparent molecular weights of purified or partially purified expression products may vary.

The use of a peptide tag in combination with the methods and compositions of the disclosure is also contemplated. A tag takes advantage of an interaction between two polypeptides. A portion of one of the polypeptides that is involved in the interaction may used as a tag. For instance, the binding region of glutathione S transferase (GST) may be used as a tag such that glutathione beads can be used to enrich for a compound containing the GST tag. An epitope tag, which an amino acid region recognized by an antibody or T cell receptor, may be used. The tag may be encoded by a nucleic acid segment that is operatively linked to a nucleic acid segment encoding a modified protein such that a fusion protein is encoded by the nucleic acid molecule. Other suitable fusion proteins are those with .beta.-galactosidase, ubiquitin, hexahistidine (6xHis), or the like. Furthermore, in some embodiments, MANF or biologically functional fragments thereof are tagged with a fluorescence tag (e.g., GFP, eGFP), which are conventionally known and utilized in detection and monitoring of proteins.

### Pharmaceutical Compositions

Pharmaceutical compositions of the therapeutic proteins described herein are within the scope of the present disclosure. In one aspect of the disclosure, pharmaceutical compositions comprising MANF or MANF and one or more additional therapeutic agents are administered to a subject to effect prophylactic or therapeutic effect. Such compositions comprise a therapeutically or prophylactically effective amount of MANF or functional fragments thereof. Examples of such additional one or more therapeutic agents include but are not limited to proteins that are or nucleic acids encoding proteins that are, "Neurotrophic factors". Neurotrophic factors are growth factors that promote differentiation, maintain a mature phenotype and provide trophic support, promoting growth and survival of neurons. Neurotrophic factors reside in the nervous system or in innervated tissues. The following have been described as neurotrophic factors: basic fibroblast growth factor, (bFGF), acidic fibroblast growth factors (aFGF), ciliary neurotrophic factor (CNTF), nerve growth factor (NGF), brain derived neurotrophic factor (BDNF), glial-derived neurotrophic factor (GDNF), neurotrophin-3 (NT3), NT4/5, insulin-like growth factor (IGF-1), IGF-II, NT-4, IL-1..beta., TNF.alpha.., transforming growth factor .beta. (TGF-.beta., TGF-.beta.1), MANF (NTN), persephin (PSP), artemin, and AL-1. Uses of neurotrophic factors are well known to those of skill in the art and can be found, for example, in U.S. Patent Publication No. 20010011126, U.S. Pat. Nos. 6,284,540, 6,280,732, 6,274,624, 6,221,676. The use of Par4 is well known to those of skill in the art, as disclosed in U.S. Pat. No. 6,111,075. In various embodiments, methods of treating a neurological disorder are contemplated within the scope of the present disclosure, where MANF or functional fragments thereof are co-administered to a subject with one or more neurogrophic factor.

In various embodiments of the disclosure, a pharmaceutical composition comprising MANF or functional fragments thereof, may further contain formulation materials for modifying, maintaining or preserving, for example, the pH, osmolarity, viscosity, clarity, color, isotonicity, odor, sterility, stability, rate of dissolution or release, adsorption or penetration of the composition. Suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates, other organic acids); bulking agents (such as mannitol or glycine), chelating agents [such as ethylenediamine tetraacetic acid (EDTA)]; complexing agents (such as caffeine, polyvinylpyrrolidone, beta-cyclodextrin or hydroxypropyl-beta-cyclodextrin); fillers; monosaccharides; disaccharides and other carbohydrates (such as glucose, mannose, or dextrins); proteins (such as serum albumin, gelatin or immunoglobulins); coloring; flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate 80, triton, tromethamine, lecithin, cholesterol, tyloxapal); stability enhancing agents (sucrose or sorbitol); tonicity enhancing agents (such as alkali metal halides (preferably sodium or potassium chloride, mannitol sorbitol); delivery vehicles; diluents; excipients and/or pharmaceutical adjuvants. (Remington's Pharmaceutical Sciences, 18th Edition, A. R. Gennaro, ed., Mack Publishing Company, 1990)..

The pharmaceutical compositions may include one or more inert excipients, which include water, buffered aqueous solutions, surfactants, volatile liquids, starches, polyols, granulating agents, microcrystalline cellulose, diluents, lubricants, acids, bases, salts, emulsions, such as oil/water emulsions, oils such as mineral oil and vegetable oil, wetting agents, chelating agents, antioxidants, sterile solutions, complexing agents, disintegrating agents and the like. Buffered solutions will typically be at physiological pH and more particularly will typically be buffered to the pH of the target tissue.

Among the preferred excipients are: water, phosphate buffered saline solutions, propylene glycol diesters of medium chain fatty acids available under the tradename Miglyol 840 (from Huls America, Inc. Piscataway, N.J.) triglyceride esters of medium chain fatty acids available under the tradename Miglyol 812 (from Huls); perfluorodimethylcyclobutane available under the tradename Vertrel 245 (from E. I. DuPont de Nemours and Co. Inc. Wilmington, Del.); perfluorocyclobutane available under the tradename octafluorocyclobutane (from PCR Gainsville, Fla.); polyethylene glycol available under the tradename EG 400 (from BASF Parsippany, N.J.); menthol (from Pluess-Stauffer International Stanford, Conn.); propylene glycol monolaurate available under the tradename lauroglycol (from Gattefosse Elmsford, N.Y.), diethylene glycol monoethylether available under the tradename Transcutol (from Gattefosse); polyglycolized glyceride of medium chain fatty adds available under the tradename Labrafac Hydro WL 1219 (from Gattefosse); alcohols, such as ethanol, methanol and isopropanol; eucalyptus oil available (from Pluses-Stauffer International): and mixtures thereof.

Compounds of the present disclosure include amino acid derivatives. A preferred surfactant may be the sodium salt form of the compound, which may include the monosodium salt form. Suitable sodium salt surfactants may be selected based on desirable properties, including high speed of polymerization, small resultant particle sizes suitable for delivery, good polymerization yields, stability including freeze-thaw and shelf-life stability, improved surface tension properties, and lubrication properties.

Surfactants which can be used to form pharmaceutical compositions and dosage forms of the invention include, but are not limited to, hydrophilic surfactants, lipophilic surfactants, and mixtures thereof. That is, a mixture of hydrophilic surfactants may be employed, a mixture of lipophilic surfactants may be employed, or a mixture of at least one hydrophilic surfactant and at least one lipophilic surfactant may be employed.

Other suitable aqueous vehicles include, but are not limited to, Ringer's solution and isotonic sodium chloride. Aqueous suspensions may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

Chelating agents which can be used to form pharmaceutical compositions and dosage forms of the disclosure include, but are not limited to, ethylene diaminetetraacetic acid (EDTA), EDTA disodium, calcium disodium edetate, EDTA trisodium, albumin, transferrin, desferoxamine, desferal, desferoxamine mesylate, EDTA tetrasodium and EDTA dipotassium, sodium metasilicate or combinations of any of these.

Lubricants which can be used to form pharmaceutical compositions and dosage forms of the disclosure include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, or mixtures thereof.

Thickening agents which can be used to form pharmaceutical compositions and dosage forms of the disclosure include, but are not limited to, isopropyl myristate, isopropyl palmitate, isodecyl neopentanoate, squalene, mineral oil, C₁₂-C₁₅ benzoate and hydrogenated polyisobutene. Particularly preferred are those agents which would not disrupt other compounds of the final product, such as non-ionic thickening agents. The selection of additional thickening agents is well within the skill of one in the art.

Other agents may also be added, such as antimicrobial agents, to prevent spoilage upon storage, i.e., to inhibit growth of microbes such as yeasts and molds. Suitable antimicrobial agents are typically selected from the group consisting of the methyl and propyl esters of p-hydroxybenzoic acid (i.e., methyl and propyl paraben), sodium benzoate, sorbic acid, imidurea, purite, peroxides, perborates and combinations thereof.

Preservatives which can be used to form pharmaceutical compositions and dosage forms of the disclosure include, but are not limited to, purite, peroxides, perborates, imidazolidinyl urea, diazolidinyl urea, phenoxyethanol, alkonium chlorides including benzalkonium chlorides, methylparaben, ethylparaben and propylparaben.

### Formulations

In various embodiments, formulations of the one or more therapeutic agents disclosed herein are within the scope of the present disclosure. For example, in various embodiments, parenteral formulations may be in the form of liquid solutions or suspensions; for oral administration, formulations may be in the form of tablets or capsules; and for intranasal formulations, in the form of powders, nasal drops, or aerosols.

Methods well known in the art for making formulations are to be found in, for example, Remington: The Science and Practice of Pharmacy, (20th ed.) ed. A. R. Gennaro AR., 2000, Lippencott Williams & Wilkins. Formulations for parenteral administration may, for example, contain as excipients sterile water or saline, polyalkylene glycols such as polyethylene glycol, oils of vegetable origin, or hydrogenated naphthalenes, biocompatible, biodegradable lactide polymer, or polyoxyethylene-polyoxypropylene copolymers may be used to control the release of the present factors. Other potentially useful parenteral delivery systems for the factors include ethylene-vinyl acetate copolymer particles, osmotic pumps, implantable infusion systems, and liposomes. Formulations for inhalation may contain as excipients, for example, lactose, or may be aqueous solutions containing, for example, polyoxyethylene-9-lauryl ether, glycocholate and deoxycholate, or may be oily solutions for administration in the form of nasal drops, or as a gel to be applied intranasally.

The present factors can be used as the sole active agents, or can be used in combination with other active ingredients, e.g., other growth factors which could facilitate dopaminergic neuronal survival in neurological diseases, or peptidase or protease inhibitors.

The concentration of the present factors in the formulations of the disclosure will vary depending upon a number of issues, including the dosage to be administered, and the route of administration.

In general terms, the factors of this disclosure may be provided in an aqueous physiological buffer solution containing about 0.1 to 10% w/v polypeptide for parenteral administration. General dose ranges are from about 1 mg/kg to about 1 g/kg of body weight per day.

In some embodiments, a dose range is from about 0.01 mg/kg to 100 mg/kg of body weight per day. The preferred dosage to be administered is likely to depend upon the type and extent of progression of the pathophysiological condition being addressed, the overall health of the patient, the make up of the formulation, and the route of administration.

The phrases "pharmaceutical or pharmacologically acceptable" refers to molecular entities and compositions that do not produce an adverse, allergic or other untoward reaction when administered to an animal, such as, for example, a human, as appropriate. The preparation of an pharmaceutical composition that contains at least one IL-1 receptor antagonist or additional active ingredient will be known to those of skill in the art in light of the present disclosure, as exemplified by Remington's Pharmaceutical Sciences, 18th Ed. Mack Printing Company, 1990. Moreover, for animal (e.g., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329. Except insofar as any conventional carrier is incompatible with the active ingredient, its use in the therapeutic or pharmaceutical compositions is contemplated.

The therapeutic agents may comprise different types of carriers depending on whether it is to be administered in solid, liquid or aerosol form, and whether it need to be sterile for such routes of administration as injection.

In various embodiments, therpeutic compositions of the disclosure can be administered intraocularly, intravenously, intradermally, intraarterially, intraperitoneally, intracranially, topically, intramuscularly, intraperitoneally, subcutaneously, intravesicularlly, mucosally, orally, topically, locally, inhalation (e.g. aerosol inhalation), injection, infusion, continuous infusion, localized perfusion bathing target cells directly, via a catheter, via a lavage, in cremes, in lipid compositions (e.g., liposomes), or by other method or any combination of the forgoing as would be known to one of ordinary skill in the art (see, for example, Remington's Pharmaceutical Sciences, 18th Ed. Mack Printing Company, 1990.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The preparation of highly concentrated compositions for direct injection is also contemplated, where the use of DMSO as solvent is envisioned to result in extremely rapid penetration, delivering high concentrations of the active agents to a small area.

The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein.

An approach for stabilizing solid protein formulations of the invention is to increase the physical stability of purified, e.g., lyophilized, protein. This will inhibit aggregation via hydrophobic interactions as well as via covalent pathways that may increase as proteins unfold. Stabilizing formulations in this context often include polymer-based formulations, for example a biodegradable hydrogel formulation/delivery system. As noted above, the critical role of water in protein structure, function, and stability is well known. Typically, proteins are relatively stable in the solid state with bulk water removed. However, solid therapeutic protein formulations may become hydrated upon storage at elevated humidity or during delivery from a sustained release composition or device. The stability of proteins generally drops with increasing hydration. Water can also play a significant role in solid protein aggregation, for example, by increasing protein flexibility resulting in enhanced accessibility of reactive groups, by providing a mobile phase for reactants, and by serving as a reactant in several deleterious processes such as beta-elimination and hydrolysis.

Protein preparations containing between about 6% to 28% water are the most unstable. Below this level, the mobility of bound water and protein internal motions are low. Above this level, water mobility and protein motions approach those of full hydration. Up to a point, increased susceptibility toward solid-phase aggregation with increasing hydration has been observed in several systems. However, at higher water content, less aggregation is observed because of the dilution effect.

In particular embodiments, prolonged absorption of an injectable composition can be brought about by the use in the compositions of agents delaying absorption, such as, for example, aluminum monostearate, gelatin or combinations thereof.

In various embodiments, formulations comprising therapeutic agents of the invention comprise a stabilizing or delivery vehicle. The term "vehicle" in this context refers to a molecule that prevents degradation and/or increases half-life, reduces toxicity, reduces immunogenicity, or increases biological activity of a therapeutic protein. Exemplary vehicles include an Fc domain as well as a linear polymer (e.g., polyethylene glycol (PEG), polylysine, dextran, etc.); a branched-chain polymer (See, for example, U.S. Pat. No. 4,289,872 to Denkenwalter et al., issued Sep. 15, 1981; U.S. Pat. No. 5,229,490 to Tam, issued Jul. 20, 1993; WO 93/21259 by Frechet et al., published 28 Oct. 1993); a lipid; a cholesterol group (such as a steroid); a carbohydrate or oligosaccharide; or any natural or synthetic protein, polypeptide or peptide that binds to a salvage receptor.

In one embodiment, this disclosure provides for at least one peptide to be attached to at least one vehicle (F₁, F₂) through the N-terminus, C-terminus or a side chain of one of the amino acid residues of the peptide(s). Multiple vehicles may also be used; e.g., Fc's at each terminus or an Fc at a terminus and a PEG group at the other terminus or a side chain.

An Fc domain is one preferred vehicle. The Fc domain may be fused to the N or C termini of the peptides or at both the N and C termini. See, for example WO 97/34631 and WO 96/32478. In such Fc variants, one may remove one or more sites of a native Fc that provide structural features or functional activity not required by the fusion molecules of this disclosure. One may remove these sites by, for example, substituting or deleting residues, inserting residues into the site, or truncating portions containing the site. The inserted or substituted residues may also be altered amino acids, such as peptidomimetics or D-amino acids. Fc variants may be desirable for a number of reasons, several of which are described below.

An alternative vehicle would be a protein, polypeptide, peptide, antibody, antibody fragment, or small molecule (e.g., a peptidomimetic compound) capable of binding to a receptor or target molecule on a target cell. For example, one could use as a vehicle a polypeptide as described in U.S. Pat. No. 5,739,277, issued Apr. 14, 1998 to Presta et al. Peptides could also be selected by phage display for binding to the FcRn salvage receptor. Such salvage receptor-binding compounds are also included within the meaning of "vehicle" and are within the scope of this disclosure. Such vehicles should be selected for increased half-life (e.g., by avoiding sequences recognized by proteases) and decreased immunogenicity (e.g., by favoring non-immunogenic sequences, as discovered in antibody humanization).

In some embodiments, a vehicle is polyethylene glycol (PEG). The PEG group may be of any convenient molecular weight and may be linear or branched. The average molecular weight of the PEG will preferably range from about 2 kiloDalton ("kDa") to about 100 kDa, more preferably from about 5 kDa to about 50 kDa, most preferably from about 5 kDa to about 10 kDa. In another embodiment, the average molecular weight of the PEG will be from about 10kDa to about 20kDa, or 20kDa to 30kDa or 30kDa to 40kDa or 40kDa to 50kDa. The PEG groups will generally be attached to the compounds of the invention via acylation or reductive alkylation through a reactive group on the PEG moiety (e.g., an aldehyde, amino, thiol, or ester group) to a reactive group on the inventive compound (e.g., an aldehyde, amino, or ester group).

A useful strategy for the PEGylation of synthetic peptides consists of combining, through forming a conjugate linkage in solution, a peptide and a PEG moiety, each bearing a special functionality that is mutually reactive toward the other. The peptides can be easily prepared with conventional solid phase synthesis as known in the art. The peptides are "preactivated" with an appropriate functional group at a specific site. The precursors are purified and fully characterized prior to reacting with the PEG moiety. Ligation of the peptide with PEG usually takes place in aqueous phase and can be easily monitored by reverse phase analytical HPLC. The PEGylated peptides can be easily purified by preparative HPLC and characterized by analytical HPLC, amino acid analysis and laser desorption mass spectrometry.

Polysaccharide polymers are another type of water soluble polymer which may be used for protein modification. Dextrans are polysaccharide polymers comprised of individual subunits of glucose predominantly linked by a1-6 linkages. The dextran itself is available in many molecular weight ranges, and is readily available in molecular weights from about 1 kDa to about 70 kDa. Dextran is a suitable water-soluble polymer for use in the present disclosure as a vehicle by itself or in combination with another vehicle (e.g., Fc). See, for example, WO 96/11953 and WO 96/05309. The use of dextran conjugated to therapeutic or diagnostic immunoglobulins has been reported; see, for example, European Patent Publication No. 0 315 456. Dextran of about 1 kDa to about 20 kDa is preferred when dextran is used as a vehicle in accordance with the present invention.

### Dosage

The actual dosage amount of a composition of the present invention administered to an animal can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

In certain embodiments, pharmaceutical compositions may comprise, for example, at least about 0.1% of an active compound. In other embodiments, the an active compound may comprise between about 2% to about 75% of the weight of the unit, or between about 25% to about 60%, for example, and any range derivable therein. In other non-limiting examples, a dose may also comprise from about 1 nanogram/kg/body weight, about 5 nanogram/kg/body weight, about 10 nanogram/kg/body weight, about 50 nanogram/kg/body weight, about 100 nanogram/kg/body weight, about 200 nanogram/kg/body weight, about 350 nanogram/kg/body weight, about 500 nanogram/kg/body weight, 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 500 milligram/kg/body weight, etc., can be administered, based on the numbers described above.

In further embodiments, the composition may comprise various antioxidants to retard oxidation of one or more component. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof.

In additional embodiments of the disclosure, methods of treatment include administration of therapeutic agents of the invention (e.g., MANF, or combinations of MANF and one or more additional neurotrophic factors) a certain time period, such as a therapeutically effective time period. In some embodiments, the administration formulations of the invention or a nucleic acid encoding MANF or biologically functional fragments is specifically contemplated. It is contemplated that the time period, in some embodiments, is within one hour of the time of the spinal cord injury to 72 hours after the spinal cord injury. In additional embodiments, the time period begins within about 1 hour of injury to 72 hours after the spinal cord injury. In still further embodiments, the time period is between 24 hours and 72 hours in length or is between 3 and 6 days in length. It is contemplated that the treatment may be administered within or after 1, 2, 3, 4, 5, 6, 7, 8, 9, 10, 11, 12, 13, 14, 15, 16, 17, 18, 19, 20, 21, 22, 23, 24 hours or 1 2, 3, 4, 5, 6, 7 days or 1, 2, 3, 4, 5 weeks, or 1, 2, 3, 4, 5, 6, 7, 8, 9 10, 11, 12 months the occurrence of the injury. Also, it is contemplated that the treatment may be administered for 1, 2, 3, 4, 5, 6, 7, 8, 9, 10, 11, 12, 13, 14, 15, 16, 17, 18, 19, 20, 21, 22, 23, 24 hours or 1 2, 3, 4, 5, 6, 7 days or 1, 2, 3, 4, 5 weeks, or 1, 2, 3, 4, 5, 6, 7, 8, 9 10, 11, 12 months or more. Chronic administration is contemplated for 5, 10, 15, 20, 25, 30, 35, 40, 45, 50, 55, minutes or 1, 2, 3, 4, 5, 6, 7, 8, 9, 10, 11, 12, 13, 14, 15, 16, 17, 18, 19, 20, 21, 22, 23, 24 or more hours. Multiple administrations are also contemplated. In some embodiments the administration is given at least twice (repeated at least once).

A subject may be administered different amounts of therapeutic agents. In some embodiments, the amount of a formulation of the disclosure (e.g., protein or nucleic acid) that is administered is between 1 and 1000 nanograms per kilogram body weight per hour. In other embodiments, the amount is between 1 and 100 nanograms per kilogram body weight per hour or between 1 and 10 nanograms per kilogram body weight per hour. It is contemplated that dosages maybe 1, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1100, 1200, 1300, 1400, 1500, 1600, 1700, 1800, 1900, 2000, 2500, 3000, 3500, 4000, 4500, 5000 nanograms per kilogram body weight per hour (ng/kg/hr). It is also contemplated that dosages may also be at least and/or not more than those same amounts.

### Gene Transfer

As noted above, gene therapy is another potential therapeutic approach in which normal copies of the gene encoding a MANF polypeptide (or a polynucleotide encoding MANF sense RNA) is introduced into cells to successfully produce the MANF polypeptide. The gene must be delivered to those cells in a form in which it can be taken up and encode for sufficient protein to provide effective dopaminergic neuronal survival-promoting activity.

Retroviral vectors, adenoviral vectors, adenovirus-associated viral vectors, or other viral vectors with the appropriate tropism for neural cells may be used as a gene transfer delivery system for a therapeutic MANF construct. Numerous vectors useful for this purpose are generally known (Miller, Human Gene Therapy 15-14, 1990; Friedman, Science 244:1275-1281, 1989; Eglitis and Anderson, BioTechniques 6:608-614, 1988; Tolstoshev and Anderson, Curr. Opin. Biotech. 1:55-61, 1990; Sharp, The Lancet 337: 1277-1278, 1991; Cometta et al., Nucl. Acid Res. and Mol. Biol. 36: 311-322, 1987; Anderson, Science 226: 401-409, 1984; Moen, Blood Cells 17: 407-416, 1991; Miller et al., Biotech. 7: 980-990, 1989; Le Gal La Salle et al., Science 259: 988-990, 1993; and Johnson, Chest 107: 77S-83S, 1995). Retroviral vectors are particularly well developed and have been used in clinical settings (Rosenberg et al., N. Engl. J. Med. 323: 370, 1990; Anderson et al., U.S. Pat. No. 5,399,346). Non-viral approaches may also be employed for the introduction of therapeutic DNA into the desired cells. For example, a MANF-encoding polynucleotide may be introduced into a cell by lipofection (Felgner et al., Proc. Natl. Acad. Sci. USA 84: 7413, 1987; Ono et al., Neurosci. Lett. 117: 259, 1990; Brigham et al., Am. J. Med. Sci. 298:278, 1989; Staubinger et al., Meth. Enzymol. 101:512, 1983), asialorosonucoid-polylysine conjugation (Wu et al., J. Biol. Chem. 263:14621, 1988; Wu et al., J. Biol. Chem. 264:16985, 1989); or, less preferably, micro-injection under surgical conditions (Wolff et al., Science 247:1465, 1990).

Gene transfer could also be achieved using non-viral means requiring infection in vitro. This would include calcium phosphate, DEAE dextran, electroporation, and protoplast fusion. Liposomes may also be potentially beneficial for delivery of DNA into a cell. Although these methods are available, many of these are of lower efficiency.

In the constructs described, MANF or pro-MANF cDNA expression can be directed from any suitable promoter (e.g., the human cytomegalovirus (CMV), simian virus 40 (SV40), or metallothionein promoters), and regulated by any appropriate mammalian regulatory element. For example, if desired, enhancers known to preferentially direct gene expression in neural cells may be used to direct MANF polypeptide expression. The enhancers used could include, without limitation, those that are characterized as tissue- or cell-specific in their expression.

RNA molecules may be modified to increase intracellular stability and half-life. Possible modifications include, but are not limited to, the addition of flanking sequences at the 5' and/or 3' ends of the molecule or the use of phosphorothioate or 2' O-methyl rather than phosphodiesterase linkages within the backbone of the molecule. This concept can be extended in all of these molecules by the inclusion of nontraditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

### Combinatorial

Furthermore, any MANF-based therapeutic is combined with one or more additional therapeutic agent including but not limited to MANF2 (see, U.S. Patent Application Publication No. 20060084619), CDNF, interferon gamma, nerve growth factor, epidermal growth factor (EGF), basic fibroblast growth factor (bFGF), neurogenin, brain derived neurotrophic factor (BDNF), thyroid hormone, bone morphogenic proteins (BMPs), leukemia inhibitory factor (LIF), sonic hedgehog, glial cell line-derived neurotrophic factor (GDNFs), vascular endothelial growth factor (VEGF), interleukins, interferons, stem cell factor (SCF), activins, inhibins, chemokines, retinoic acid and ciliary neurotrophic factor (CNTF) or a combination thereof. Examples of additional therapeutic agents and methods useful with the methods and compositions of the invention are disclosed in U.S. Patent Application Nos: 20080057028; 20070082848, 20070077649

The compositions may be administered alone or in combination with at least one other agent, such as stabilizing compound, which may be administered in any sterile, biocompatible pharmaceutical carrier, including, but not limited to, saline, buffered saline, dextrose, and water. The compositions may be administered to a patient alone, or in combination with other agents, drugs or hormones.

While human MANF is preferred for use in the methods described herein, MANF has been identified in numerous species, including rat, mouse, and cow. One in the art will recognize that the identification of MANF from other animals can be readily performed using standard methods. Any protein having dopaminergic neuronal survival-promoting activity and encoded by a nucleic acid that hybridizes to the cDNA encoding human ARP is considered part of the invention.

The following examples are to illustrate the invention.

### EXAMPLE 1 :Production and Analysis of VMCL-1 Cells

The VMCL-1 cell line was made as follows. Rat E14 mesencephalic cells, approximately 2-3% of which are glioblasts, were incubated in medium containing 10% (v/v) fetal bovine serum for 12 hours and subsequently expanded in a serum-free medium, containing basic fibroblast growth factor (bFGF) as a mitogen. After more than 15 DIV, several islets of proliferating, glial-like cells were observed. Following isolation and passaging, the cells (referred to herein as VMCL-1 cells) proliferated rapidly in either a serum-free or serum-containing growth medium. Subsequent immunocytochemical analysis showed that they stained positive for two astrocytic markers, GFAP and vimentin, and negative for markers of oligodendroglial or neuronal lineages, including A2B5, 04, GalC and MAP2. We have deposited the VMCL-1 cell line with the ATCC (Accession No: PTA-2479; deposit date: Sep. 18, 2000).

Serum-free CM, prepared from the VMCL-1 cells, caused increased survival and differentiation of E14 mesencephalic dopaminergic neurons in culture. These actions are similar to those exerted by CM derived from primary, mesencephalic type-1 astrocytes. The expression of mesencephalic region-specific genes (e.g., wnt-1, en-1, en-2, pax-2, pax-5 and pax-8), was similar between VMCL-1 cells and primary, type-1 astrocytes of E14 ventral mesencephalic origin. In both, wnt-1 was expressed strongly, and en-1 less strongly, supporting an expression pattern expected of their mesencephalic origin. A chromosomal analysis showed that 70% of the cells were heteroploid, and of these, 50% were tetraploid. No apparent decline in proliferative capacity has been observed after more than twenty-five passages. The properties of this cell line are consistent with those of an immortalized, type-1 astrocyte.

The VMCL-1 cells have a distinctly non-neuronal, glial-like morphology, but lack the large, flattened shape that is typical of type-1 astrocytes in culture. Immunocytochemical analysis demonstrated that they stained positive for GFAP and vimentin, and negative for MAP2, A2B5 and 04. The cells were therefore not of the oligodendrocyte lineage. On the basis of a negative reaction to A2B5 and their morphological characteristics they were also not type-2 astrocytes. The classification that is supported by the immunocytochemical evidence is of type-1 astrocytes, although, as noted, these cells lack the classical morphological traits of primary type-1 astrocytes in culture.

### EXAMPLE 2: Action of VMCL-1 CM on E14 Dopaminergic Neurons in Culture

VMCL-1 CM was tested at 0, 5, 20 and 50% v/v, for its ability to influence survival and development of E14 mesencephalic dopaminergic neurons in culture. The cultures were primed with 10% fetal bovine serum (FBS) for 12 hours, then grown in a serum-free growth medium thereafter, until they were stained and analyzed after 7 DIV. There was a dose-dependent action of the CM on the increased survival of dopaminergic neurons. The CM increased survival by 5-fold. In contrast, there was no significant increase in non-dopaminergic neuronal survival. The profile of the biological action of this putative factor is quite different from that of CM derived from the B49 glioma cell line, the source of GDNF (Lin et al., Science 260: 1130-1132).

### EXAMPLE 3: Gene Expression Analysis of VMCL-1 Cells

To further investigate the similarity between the VMCL-1 cell line and primary cultured astrocytes, we measured the expression of six marker genes characteristic of the mesencephalic region. Analysis of wnt-1, en-1, en-2, pax-2, pax-5, and pax-8 showed that all genes were expressed in both E13 and E14 ventral mesencephalon neural tissue, with the exception of pax-2, which was expressed at E13 but not E14 neural tissue. Both primary astrocytes and VMCL-1 cells expressed wnt-1 at levels comparable with those of E13 and E14 ventral mesencephalic neural tissue. The degree of expression of en-1 was similar in primary astrocytes and VMCL-1 cells, although at a lower level versus expression in E13 and E14 ventral mesencephalic tissue. In contrast, en-2, pax-5 and pax-8 were not expressed in either primary astrocytes or VMCL-1. Pax-2 was expressed in E13 but not E14 ventral mesencephalon, and in primary astrocytes, but not in VMCL-1. EXAMPLE 4: Chromosomal Analysis of VMCL-1 Cells

Chromosomes were counted in 34 cells. Of these, 9 had a count of 42, the diploid number for rat. Of the 25 cells that were heteroploid, 12/25 or 48% were in the tetraploid range. Hyperdiploid (counts of 43-48) and hypodiploid (counts of 39-41) cells each accounted for 20% of the population, while 12% of the cells had structurally rearranged chromosomes.

The selective action of VMCL-1 CM in increasing the survival of dopaminergic neurons in culture provides a potential clinical use for the molecule(s) produced by this cell line. The lack of a toxic action of VMCL-1 CM at a concentration of 50% v/v indicates that the active, putative neurotrophic factor is not toxic. The action exerted by VMCL-1 CM mirrors almost exactly that of CM prepared from mesencephalic, primary type-1 astrocytes. A high degree of specificity of the putative factor from VMCL-1 for dopaminergic neurons is strongly indicated from the observation that general neuronal survival was not significantly increased, while the survival of dopaminergic neurons was increased 5-fold. It was demonstrated that primary type-1 astrocytes express GDNF mRNA, but have not detected GDNF protein by Western blot in the CM, at a sensitivity of 50 pg. Moreover, it was shown that under the present experimental conditions, the increased survival of dopaminergic neurons mediated by an optimal concentration of GDNF is never greater than 2-fold. These observations alone indicate that the factor responsible for the neurotrophic actions of VMCL-1 CM is not GDNF.

### EXAMPLE 5: Production of Type-1 Astrocyte-Conditioned Medium

E16 type-1 astrocyte CM (10 L) was filtered and applied to a heparin sepharose CL-6B column (bed volume 80 mL) which had previously been equilibrated with 20 mM Tris-HCl (Mallinckrodt Chemical Co. Paris, Ky.) pH 7.6 containing 0.2 M NaCl. After washing with equilibration buffer, bound proteins were eluted from the column with a linear gradient of 0.2 M-2 M NaCl in 20 mM Tris-HCl pH 7.6 (400 mL total volume, flow rate 100 mL/hr). Fractions were collected using a Pharmacia LKB fraction collector and absorbance was measured at 280 nm (Sargent-Welch PU 8600 UV/VIS Spectrophotometer). A 1 mL aliquot was taken from each fraction, pooled into groups of four (4 mL total volume) and desalted using Centricon-10.RTM. membrane concentrators (Millipore, Bedford, Mass.). Samples were diluted 1:4 in defined medium and bioassayed for dopaminergic activity. Active fractions were pooled (80 mL total volume) and then applied to a G-75 Sephadex.RTM. column (70x2.5 cm, Pharmacia Biotechnology Ltd., Cambridge, UK) which had been pre-equilibrated with 50 mM ammonium formate pH 7.4. Proteins were separated with the same buffer (flow rate, 75 mL/hr) and absorbance was measured at 280 nm. A 1 mL aliquot was taken from each fraction, pooled into groups of four (4 mL total volume), concentrated by lyopholyzation and reconstituted in 1 mL distilled water volume. Samples were then diluted 1:4 in defined medium for dopaminergic bioassay. Those with neurotrophic activity were further bioassayed as individual fractions.

An important distinguishing feature of VMCL-1 CM is that it promotes predominantly the survival of dopaminergic neurons, compared with the survival of GABAergic, serotonergic, and other neuronal phenotypes present in the culture. This claim of specificity is also made for GDNF. The results of extensive testing have demonstrated, however, that the VMCL-1-derived compound is not GDNF.

### EXAMPLE 6: Isolation and Purification of a Protein having Dopaminergic Neuronal Survival-Promoting Activity

The purification protocol was performed as follows. All salts used were of the highest purity and obtained from Sigma Chemical Co. All buffers were freshly prepared and filtered via 0.2.mu.M filter (GP Express vacuum-driven system from Millipore)

### Step 1: Heparin-Sepharose Column Chromatography (4° C.)

Three liters of VMCL-1 conditioned medium was diluted with an equal volume of 20 mM sodium phosphate buffer, pH 7.2 at room temperature, filtered, and concentrated to 550 mL volume with 5K PREP/SCALE - TFF 2.5 ft² cartridge (Millipore). The concentrated material was loaded onto a 10 mL Heparin-Sepharose column assembled from 2x5 mL HiTrap Heparin columns (Pharmacia Biotech) and pre-equilibrated with at least 100 mL of 10 mM sodium phosphate buffer, pH 7.2 (buffer A). After the loading was complete, the column was washed with 100 mL of buffer A. A total of 10 fractions were eluted with buffer B (buffer A plus 1 M sodium chloride) in about 3 mL volumes each. A 300 L sample was withdrawn for analysis.

### Step 2: Superose 12 Column Chromatography (4° C.)

All of the fractions from step 1 were pooled, then concentrated to 4.5 mL using Centricon Plus-20 concentrator (5,000 MWCO, Millipore), loaded onto 16x600 mm gel-filtration column packed with Superose 12 media (Prep Grade, Sigma Chemical Co.) and pre-equilibrated with at least 300 mL of 20 mM sodium phosphate buffer, pH 7.2 containing 0.6 M sodium chloride (GF buffer). The protein elution was conducted in GF buffer. Two milliliter fractions were collected and analyzed for activity. The active protein was eluted in a 15 mL volume after 84 ml of GF buffer was passed through the column and corresponded to an approximately 20-30 kDa elution region based on the column calibration data obtained with protein standards (Bio-Rad).

### Step 3: Ceramic Hydroxyapatite Column Chromatography (Room Temperature; FPLC System)

The active fractions from step 2 that corresponded to the 20-30 kDa elution region were pooled and concentrated to 7.5 mL, using a Centricon Plus-20 concentrator (5,000 MWCO), dialyzed overnight at 4° C. against 2 L of 10 mM sodium phosphate buffer, pH 7.2 (buffer A) and loaded (via Superloop) onto a 1 mL pre-packed ceramic hydroxyapatite (Type I, Bio-Rad) column equilibrated with buffer A. After the excess of unbound protein (flow through) was washed off the column with buffer A, the linear gradient of buffer A containing 1.0 M NaCl was applied from 0 to 100%. One milliliter fractions were collected and analyzed for activity. The active protein was eluted as a broad peak within the region of gradient corresponding to 0.4-0.8 M NaCl concentration.

### Step 4: Anion-Exchange Column Chromatography (Room Temperature; FPLC System)

The fractions corresponding to the broad peak were pooled (total volume=15 mL) and concentrated to 6 mL using Centricon Plus-20 (5,000 MWCO), dialyzed overnight at 4° C. against 2 L of 20 mM Tris HCl buffer, pH 7.5 (buffer A), loaded (via Superloop) onto a 1 mL anion-exchange FPLC column (Uno, Bio-Rad), and equilibrated with buffer A. After the excess of unbound protein was washed off the column with buffer A, a linear gradient of 0-100% 1 M NaCl (in buffer A) was applied. One milliliter fractions were collected and analyzed for activity. The active protein was found in the flow-through (i.e., in the unbound protein fraction).

### Step 5: BioSil 125 Column Chromatography (Room Temperature; HPLC System)

The active protein fraction from Step 4 (7 mL of total volume) was concentrated down to nearly zero volume (about 1 .mu.L) using Centricon Plus-20 concentrator (5,000 MWCO) and reconstituted in 0.6 mL of 10 mM sodium phosphate buffer, pH 7.2. The reconstituted material (70 .mu.L, analytical run) was loaded onto BioSil 125 HPLC gel-filtration column (Bio-Rad) equilibrated with 20 mM sodium phosphate buffer, pH 7.2 (GF buffer). The chromatography was conducted using HP 1100 Series HPLC system (Hewlett-Packard). The eluate was collected in 120 .mu.L fractions and analyzed for activity and protein content (SDS-PAGE). The activity was found in fractions associated with the main 280-nm absorbance peak eluted from the column, which was represented by a 45-kDa protein according to SDS-PAGE analysis. Nevertheless, no activity was found in the side fractions of the 45-kDa protein peak, indicating that activity might be due to the presence of another protein that was co-eluted with 45 kDa protein, but at much lower concentration that could not be detected on the 12% SDS-PAGE silver-stained gel. Therefore, the remaining concentrated material from step 5 was further concentrated down to 80 .mu.L volume using a Centricon-3 concentrator (Millipore), and 60 .mu.L was loaded and separated on the column at the same conditions as for the above-described analytical run. Aliquots of 8 .mu.L were taken from each 120 .mu.L fraction of the eluate and analyzed by SDS-PAGE (12% gel) combined with silver staining. This analysis indicated that another two additional proteins (having molecular weights of about 18 and 20 kDa) were associated with the active fractions and co-eluted with the major 45-kDa protein. The active fractions were dialyzed against 1 L of ammonium acetate buffer, pH 8.0 (4° C.) and combined to create two active pools, P-1 and P-2, such that P-1 contained the 20 kDa protein and the 45 kDa protein, and P-2 contained the 18 kDa protein and the 45 kDa protein. Each pool was dried down on SpeedVac vacuum concentrator (Savant) and separately reconstituted in 15 .mu.L 0.1 M ammonium acetate buffer, pH 6.9. Aliquots were withdrawn from each sample and assayed for activity. Additionally, 1 .mu.L aliquots were subjected to 12% SDS-PAGE analysis followed by silver staining.

The results of the foregoing analysis clearly indicated that P-1, but not P-2, contained the desired survival-promoting activity. In the next step, both P-1 and P-2 were dried on SpeedVac, reconstituted (each) in 10 .mu.L of freshly prepared SDS-PAGE reducing sample buffer (Bio-Rad), incubated for one minute in a boiling water bath and loaded onto a 12% SDS-PAGE gel. After electrophoresis was complete, the gel was fixed in methanol/acetic acid/water solution (50:10:40) for 40 minutes at room temperature, washed three times with nanopure water, and stained overnight with GelCode Blue Stain Reagent (Pierce) at room temperature. After staining was completed, and the GelCode solution was washed off the gel with nanopure water, the visible protein bands corresponding to the 45 kDa protein (both P-1 and P-2) and the 20 kDa protein (P-1 only) were excised from the gel with a razor blade. Each gel slice containing a corresponding band was placed in a 1.5 mL microcentrifuge tube until the time of in-gel digestion.

### EXAMPLE 7: Analysis of In-Gel Digested Fragments by nESI-MS/MS

The protein gel bands were incubated with 100 mM ammonium bicarbonate in 30% acetonitrile (aq.) at room temperature for 1 hour in order to remove the colloidal comassie blue stain. The destaining solution was replaced a number of times until the dye was completely removed. The gel pieces were then covered with deionized water (.about.200 .mu.L) and shaken for 10 minutes. The gel pieces were dehydrated in acetonitrile and, after removing the excess liquid, were dried completely on a centrifugal evaporator. The gel bands were rehydrated with 20 .mu.L of 50 mM ammonium bicarbonate, pH 8.3, containing 200 ng of modified trypsin (Promega, Madison, Wis.). The gel pieces were covered with 50 mM ammonium bicarbonate, pH 8.3 (approximately 50 .mu.L), and were incubated overnight at 37° C. The digest solutions were then transferred to clean eppendorf tubes and the gel pieces were sonicated for 30 minutes in 50-100 .mu.L of 5% acetic acid (aq). The extract solutions were combined with the digest solutions and evaporated to dryness on a centrifugal evaporator.

The in-gel digest extracts were first analyzed by matrix-assisted laser desorption ionization-time of flight mass spectrometry (MALDI-TOFMS) using a Voyager Elite STR MALDI-TOFMS instrument (Applied Biosystems Inc., Framingham, Mass.). The extracts were dissolved in 5 .mu.L of 50% acetonitrile, 1% acetic acid. Dihydroxybenzoic acid was used as the matrix and spectra were acquired in positive ion, reflectron mode. Approximately one fifth of each sample was used for this analysis. These spectra provided the masses of the peptides in the digest extracts which were then used to search an in-house, non-redundant protein sequence database, a process called peptide mass fingerprinting. The remainder of the samples were used for peptide sequencing analysis by nanoelectrospray ionization-tandem mass spectrometry (nESI-MS/MS). The extracts were first desalted using C18 ZipTips (Millipore) and redissolved in 75% methanol (aq.), 0.1% acetic acid (5 .mu.L). Approximately one half of the samples were loaded into nanoelectrospray glass capillaries (Micromass). nESI-MS/MS analyses were carried out using a Q-Star quadrupole time-of-flight hybrid mass spectrometer (PE SCIEX, Concord, ON). All MS/MS analyses were carried out in positive ion mode. The collision gas was nitrogen and the collision energy was 40-60 eV. MS/MS spectra were typically acquired every second over a period of two minutes. The MS/MS spectra were used to search an in-house non-redundant protein sequence database using partial sequence tags (i.e., only the peptide mass and a few fragment ions are used to search the database). If the protein was not identified by this procedure then the amino acid sequences of two or more peptides were determined as fully as possible from the MS/MS spectra. These sequences were used to carry out BLAST searches on NCBI's protein, nucleotide and EST sequence databases.

### EXAMPLE 8: Identification of MANF, a Secreted Form of ARP

In order for ARP to be a factor that is responsible at least in part for the observed neurotrophic activity of VMCL-1 CM, the protein must be released from the cell. The predicted amino terminus of ARP has basic charges, however, a property that would favor retention in the cell nucleus. Nonetheless, we hypothesized that there would also exist a secreted form.

In a publication by Goo et al. (Molecules and Cells 9:564-568, 1999), a cDNA encoding an ARP-like protein in Drosophila melanogaster was identified while screening a cDNA library using a yeast signal sequence trap technique. The putative ARP-like protein encoded by this cDNA lacks the arginine-rich amino terminus. Using the SignalP program, we identified a signal peptide (residues 1-22) and a signal peptidase-cutting site between alanine 22 and leucine 23 of Drosophila ARP-like protein, providing additional evidence that Drosophila ARP-like protein is secreted.

Based on the alignment between human ARP and Drosophila ARP-like protein, we postulated that human ARP would have a signal sequence and signal peptidase cutting site. Accordingly, we used the SignalP program to analyze the human ARP lacking the arginine-rich amino terminus (amino acids 1-55); this polypeptide is now referred to as pro-MANF. In this example, the methionine at position 56 is the start codon. The SignalP program predicted a signal peptide consisting of residues 1-21 of SEQ ID NO: 2 and a cutting site between alanine 21 and leucine 22, which is consistent with the results from the analysis of the Drosophila ARP-like protein.

Based in part on our analysis of Drosophila ARP-like protein (GenBank Accession No. AF132912₋₋₁) and human ARP, we predict that the translation can begin at either the methionine at position 1 or the methionine at position 56 of human ARP. In the latter case, the signal peptide-containing protein (pro-MANF) is capable of being secreted from the cell in the form of MANF, where the protein acts a neurotrophic factor. Our discovery of the existence of MANF does not, however, preclude an intracellular function for the ARP containing the arginine-rich amino-terminal region.

### EXAMPLE 9: Biological Activity of MANF Expressed in E. coli

Recombinant protein expression was carried out in E. coli bacterial cells using pTriEx containing a polynucleotide encoding human MANF. A total of 4 mg of purified recombinant MANF was obtained from 350 mL of bacterial cell culture, its identity confirmed by mass spec sequencing. This protein was tested for its ability to protect DA neurons. MANF expressed in E. coli was capable of protecting DA neurons from cell death to the same extent as did the VMCL-1 conditioned medium.

### EXAMPLE 10: Dose-Response for Eukaryotic MANF Expressed in HEK293 Cells

The dose-relationship of human MANF (99% pure, produced in HEK293 cells) versus survival of dopaminergic neurons was tested using a dopaminergic cell culture assay system containing 20% of dopaminergic neurons. E14 pregnant rats were killed by CO₂ narcosis. The torso was soaked in 70% EtOH, a laporatomy was performed, and the uterine sac removed and transferred to a 50 mL tube containing 20 mL cold HBSS, pH 7.4. Each uterine sac was in turn transferred to a 10-cm petri dish containing 15 mL cold HBSS. The fetuses were removed intact, and each brain was isolated intact and transferred to a new 10-cm petri dish containing 15 mL cold HBSS. The medial ventral mesencephalon (VM) at the roof of the mesencephalic flexure was dissected to obtain 1.0 mm³ piece of tissue at a packing density of 1.0x1 cells/mm³. The VM tissue was transferred to a 15 mL tube containing 10 mL of cold PCM10. The pooled VM tissue was washed with PCM10 (DMEM/F12 with 2 mM glutamine, 5 mg/mL insulin, 5 mg/mL transferrin, 5 mg/mL sodium selenite; 20 nM progesterone, 30 nM thyroxine, and 10% fetal bovine serum) (three washes), followed by a single wash in serum-free medium (PCM0; same as PCM10 except that it lacks fetal bovine serum) and digested in 2.0 ml of PCMO containing papain (10 U/mL) for 15 minutes, at 37° C. The tissue was then rinsed (3x5 mL) with PCM10, to inactivate the protease activity. Trituration was done in 2.0 mL of PCM0, using a P-1000 set at 500 .mu.L. The end point is a milky suspension with no signs of tissue clumps. The dispersed cells were centrifuged (1,000 rpm, 2 min, 4° C.), counted, then resuspended at a density of 6.25x10⁵ cells/mL in PCM10. Cell viability was tested at this stage, and was usually >95%.

The cells were plated as microisland (MI) droplets of 25 .mu.L, (1.56x10⁴ cells/MI) on 8-well chamber slides, coated with poly-D-lysine. A 25 .mu.L MI droplet occupies an area of 12.5 mm². The average, final, mean cell density of the MI is therefore 1.25x10⁵ cells/cm². The mean cell density at the center of the MI is about 2.0x10⁵/cm², falling off to <1.0x10⁴ at the periphery of the MI. The MIs were incubated at 37° C., in 5% CO₂ at 100% humidity for 45 minutes to allow the cells to attach to the coated surface. After attachment, 375 .mu.L of PCM10 was added to each well, and the cells serum-primed for 4 hr. At the end of priming, 100% of PCM10 was aspirated, and replaced with serum-free, PCM0.

MANF was prepared as follows. Human pro-MANF was cloned into a pTriEx expression vector. Recombinant protein expression was carried out in HEK293 cells. Twenty micrograms of purified recombinant MANF lacking the signal sequence) was obtained from 800 mL of HEK293 cell conditioned medium. Its identity was confirmed by mass spec sequencing analysis.

Cultures were treated on the first, third and fifth days with the indicated amount of MANF. The cultures were fixed and stained on DIV6 or DIV7, using either the Vector ABC method, or indirect immunofluorescence.

As early as DIV3, there was a significant difference between the different concentrations of MANF tested (ANOVA, P<0.001). Paired comparisons using the Tukey method of analysis, indicated that MANF at 250 and 500 pg/ml and 1.0 and 10 ng/nL were significantly different from controls (P<0.05).

### EXAMPLE 11: Rank Order of Potency among BDNF, GDNF and MANF

When three equivalent doses of BDNF, GDNF and MANF were tested, the rank order of potency was: MANF>GDNF>BDNF, indicating that the two lower concentrations of MANF were more selective for DA neurons, relative to low doses of BDNF or GDNF. At the highest dose, the rank order of potency was: GDNF>MANF>BDNF. In general, BDNF tended to be the most potent, but least specific for protecting DA neurons. At lower concentrations, MANF tended to be the most selective in protecting DA neurons. However, in one embodiment, MANF is contemplated to be co-administered with GDNF and/or BDNF to effect an enhanced beneficial effect.

### EXAMPLE 12: Selectivity of Responsiveness to MANF

The ability of MANF to protect neurons from other brain regions was tested. No protection of rat cerebellar granule neurons, nodose sensory neurons, or sympathetic noradrenergic neurons was observed. Similarly, in ventral mesencephalic cultures, there appeared to be no activity on GABAergic and serotonergic neurons in cultures in which MANF was demonstrably protective for DA neurons. In contrast to the foregoing results, MANF was protective for a subset of dorsal root ganglion cells in culture. Dorsal root ganglia consist of at least three subpopulations of neurons. It has been demonstrated that NGF, BDNF and NT-3, all members of the neurotrophin family of neurotrophic factors, each acts on a different subset of these neurons. The action of MANF on this subset of dorsal root ganglion neurons, is therefore in keeping with the general neuroprotective proterties of neurotrophic factors.

### EXAMPLE 13: Production of MANF Polyclonal Antibodies

Polyclonal antibodies were prepared as follows. His-tagged full length MANF was prepared in E. coli. Six antigen injections of 200 ug of purified MANF protein per injection per rabbit were performed (one each on days 1, 21, 35, 49, 63, and 70). The serum was collected on day 84 (100 mL serum/rabbit).

Western blot analysis was used to test the activity of MANF-pAb. A relatively high quantity of MANF (720 ng) was used for the initial test of the activity of MANF-pAb, which remained active at a dilution of 1: 12,800. In the next test, the dilution of MANF was fixed at 5,000 and the quantity of MANF varied from 1,000 to 15.6 ng. The lowest quantity of MANF, 15.6 ng, was easily detected. In tests for cross reactivity with BDNF and GDNF, the results showed that even at three times the quantity of MANF (32 ng), the MANF-pAb did not cross react with either BDNF or GDNF. The foregoing results were obtained with the following methods.

### Mesencephalic Cultures

Primary mesencephalic cell culture was prepared from timed-pregnant Sprague-Dawley rats (Taconic Farms; Germantown, N.Y.). as described previously (Shimoda et al., Brain Res. 586:319-323, 1992 ; Takeshima et al., J. Neurosci. 14:4769-4779, 1994; Takeshima et al., Neuroscience. 60:809-823, 1994; Takeshima et al., J. Neurosci. Meth. 67:27-41, 1996). The dissected tissue was collected and pooled in oxygenated, cold (4° C.), HBSS or medium containing 10% fetal bovine serum (Biofluids Laboratories, Rockville, Md.), depending on the purpose of the experiment. Pregnant rats were killed by exposure to CO₂ on the fourteenth gestational day (i.e., E14), the abdominal region was cleaned with 70% EtOH, a laparotomy was performed, and the fetuses collected and pooled in cold Dulbecco's phosphate-buffered saline (DPBS), pH 7.4, without Ca²⁺ or Mg²⁺. The intact brain was then removed, a cut was made between the diencephalon and mesencephalon, and the tectum slit medially and spread out laterally. The ventral, medial 1.0 mm³ block of tissue comprising the mesencephalic dopaminergic region was isolated. Dissected tissue blocks were pooled in cold (4° C.), oxygenated medium. The tissue was triturated without prior digestion. Alternatively, the cells were incubated in L-15 growth medium containing papain (Sigma Chemical Co.), 10 U/mL, at 37°C., for 15 minutes, washed (3x2 mL) with medium, and triturated using only the needle and syringe. The dispersed cells were transferred to 1.5 mL Eppendorf tubes (1.0 mL/tube), and centrifuged at 600 g for 2 minutes. The use of higher centrifugation speeds for longer pexriods results in contamination of the cultures with debris and, as a result, suboptimal growth of the cells. The medium was carefully aspirated, and the cells resuspended in fresh medium and counted using a hemocytometer. All procedures, from laparotomy to plating were completed within 2 hours. In a typical experiment, one litter of 10-15 fetuses yielded 1.0x10⁵ cells/fetus, or 1.0x10⁶-1.5x10⁶ cells/litter.

### Mesencephalic Microisland Cultures

To make mesencephalic microisland cultures, cells were prepared as described above, and resuspended at a final density of 5.0x10⁵ mL. A 25 uL droplet of the suspension (1.25x10⁴ cells) was plated using a 100 .mu.L pipette onto 8-well chamber slides coated with poly-D-lysine (50 ug/mL). The area of the droplet was about 12.5 mm², for a final mean cell density of 1.0x10⁵/cm². The droplet was dispensed uniformly, and the pipette tip withdrawn vertically, to avoid smearing. The area occupied by the microisland culture remained uniform for the duration of the culture. The cultures were incubated for 30 minutes at 37° C., in 5% CO₂ at 100% humidity, to allow the cells to attach, and 375 uL of growth medium was then added to each well. The medium was changed after the first 12 hours, and approximately half of the medium was changed every second day thereafter.

### Cell Viability Assay

A two-color fluorescence cell viability assay kit (Live/Dead Viability/Cytotoxicity Assay Kits, #L-3224, Molecular Probes, Inc., Eugene, Oreg.) was used to identify live and dead cells prior to plating and in cultures. Live and dead cells fluoresced green and red, respectively, giving two positive indicators of viability. Ethidium homodimer and calcein-AM were diluted with DPBS to give final concentrations of 3.8 .mu.M and 2.0 .mu.M, respectively. Evaluation of cell viability was done before plating. A cell suspension was incubated for 15 minutes with an equal volume of dye (typically 20 .mu.L) on glass slides at room temperature in a dark, humid chamber, coverslipped, and then examined with a fluorescent microscope using FITC optics. Cell viability just before plating was about 95%.

### Culture Medium

The serum-free medium used consisted of equal volumes of Dulbecco's modified Eagle medium (DMEM) and Ham's F-12 (Gibco, Grand Island, N.Y.; 320-1320AJ), 1.0 mg/mL bovine albumin fraction V (Sigma Chemical Co.; A4161), 0.1 .mu.g/mL apo-transferrin (Sigma; T-7786), 5 .mu.g/mL insulin (Sigma; 1-1882), 30 nM L-thyroxine (Sigma; T-0397), 20 nM progesterone (Sigma; P-6149), 30 nM sodium selenite (Sigma; S-5261), 4.5 mM glutamine (Gibco, 320-5039AF), 100 U/mL penicillin, and 100 .mu.g/mL streptomycin (Gibco; P-100-1-91).

### Preparation of Conditioned Medium from VMCL-1 Cell Line

In preparing conditioned medium from the VMCL-1 cell line, 2.0x10⁶ cells were plated in a 15 cm uncoated culture dish, in 20 mL of growth medium containing 1.0% of FBS. At 80% confluence, the medium was aspirated and the cells washed once with serum-free medium. 20 mL of serum-free N2 medium without albumin was added, and conditioning allowed to continue for 48 hours. During this time, the cells usually expanded to 100% confluence. The medium was aspirated, pooled in 50 mL tubes, centrifuged (15,000 rpm for 20 minutes) and subsequently pooled in a 1.0 L plastic bottle. Usually 5 mL of each batch of CM was filter-sterilized using a 0.22 .mu.m filter, stored at aliquots of 5 mL, at -70° C., and used to determine neurotrophic potency, before being pooled with the larger store of CM. If desired, VMCL-1 CM can be made in large quantities using standard industrial cell culture techniques known to those in the art.

### Production of Conditioned Medium for Type-1 Astrocytes

Type-1 astrocytes were prepared as follows. E16 rat fetal brain stem was dissected in cold DPBS, and the mesencephalic region transferred to astrocyte culture medium (DMEM/Ham's F-12, 1:1, 15% FBS, 4.0 mM glutamine, 30 nM sodium selenite, penicillin, and streptomycin). Cells were dispersed by trituration in 2 mL of fresh medium using an 18-gauge needle fitted to a syringe. Cells were centrifuged for 5 minutes at 2,000 rpm in a centrifuge, re-suspended in medium, and triturated again. The final cell pellet was dispersed and plated at a density of 1x10⁶ cells/75 cm² flask in 15 mL of medium. Cells were incubated at 37° C. in an atmosphere of 5% carbon dioxide and 95% air for 24 hours, and unattached cells were removed by aspiration. Cells were cultured for an additional nine days, and flasks were then shaken vigorously for 16 hours to remove any contaminating cell types. Astrocyte monolayers were washed three times with DPBS, trypsinized and replated (density of 1x10⁶ cells/flask). At this time, a small proportion of the cells were plated onto eight-well chamber slides (Nunc Inc., Naperville, Ill.); these sister cultures were treated as described for the flask cultures. At confluence, the medium was replaced with medium containing 7.5% FBS and cells were incubated for 48 hours. At the next exchange, defined serum-free medium (DMEM/Ham's F-12, 1:1, 4.0 mM glutamine, 30 nM sodium selenite, penicillin 100 U/ml and streptomycin 100 U/mL) was added and cells were incubated for a further 48 hours. Medium was replaced and, after five days, conditioned medium was harvested and mixed with leupeptin (10 mM: Bachem, Torrance, Calif.) and 4-(2-aminoethyl)-benzenesulfonyl fluoride hydrochloride (1.0 mM: ICN Biochemicals, Aurora, Ohio) to inhibit proteolysis. At the time of harvesting, astrocyte monolayers cultured on chamber slides were immunostained in order to assess the culture phenotype.

### Culturing of VMCL-1 Cells

In culturing VMCL-1 and preparing VMCL-1 CM, 2.0x10⁶ cells were plated in a 15-cm uncoated culture dish, in 20 mL growth medium initially containing 10% FBS. At 80% confluence, the medium was aspirated and the cells washed once with serum-free medium. Usually 20 mL of serum-free medium without albumin was added, and conditioning allowed to continue for 48 hours. N2 medium proved to be particularly suitable for use to collect conditioned medium. During these 48 hours, the cells usually expanded to 100% confluence. The medium was aspirated, pooled in 50 mL tubes, centrifuged (15,000 rpm, 20 min) and pooled in a 1.0 L plastic bottle. Approximately 5 mL of each batch of CM was sterilized using a 0.22 mm filter, stored at aliquots of 0.5 mL, at -70° C., and used to determine neurotrophic potency, before being pooled with the larger store of CM. The VMCL-1 cell line has now been passaged greater than 50 times.

### Immunocytochemistry

For MAP2 and TH immunocytochemistry, the cultures were washed (2x250 uL) with cold DPBS, fixed with 4% formaldehyde in PBS for 10 minutes, permeabilized using 1% CH₃COOH/95% EtOH at -20° C., for 5 minutes, and then washed (3x250 uL) with PBS. Non-specific binding was blocked with 1% bovine serum albumin in PBS (BSA-PBS) for 15 minutes. Anti-TH antibody (50 uL) (Boehringer-Mannheim, Indianapolis, Ind.), or anti-MAP2 antibody (Boehringer-Mannheim) was applied to each well, and the slides incubated in a dark humid box at room temperature for 2 hours. Control staining was done using mouse serum at the same dilution as the anti-TH antibody. After washing (2x250 u L) with PBS, anti-mouse IgG-FITC (50 uL) was applied, and the slides incubated for an additional 1 hour. After washing with PBS (2x250 uL), excess fluid was aspirated, the chamber walls removed, and a single drop of VectaShield mounting medium (Vector Laboratories, Burlingame, Calif.) applied, followed by a cover glass, which was sealed with nail polish. In some experiments, TH was identified using biotinylated, secondary antibodies, and the nickel-enhanced, diaminobenzidine (DAB) reaction product was developed using the biotinylated peroxidase-avidin complex (ABC kit; Vector Laboratories).

For glial fibrillary acidic protein (GFAP, Boehringer-Mannheim, #814369), fixation and permeabilization were done in one step using 5% CH₃COOH/95% C₂H₅OH at -20° C. The subsequent procedures were the same as those used to visualize TH. For A2B5 and 04, the cultures were washed with cold DPBS (2x250 uL) and blocked with 1% BSA-PBS for 10 minutes. The A2B5 antibody (50 uL) was applied to each well, and incubated for 1 hour. After washing with DPBS (2x 250 uL), the secondary antibody, anti-IgM-FITC, was applied for 30 minutes. The cells were then washed with DPBS (2x250 uL). To counter-stain cell nuclei, cells were incubated with 0.5 .mu.g/mL of nucleic acid dye H33258 (Hoechst, Kansas City, Mo.) in 10 mM sodium bicarbonate for 15 minutes at room temperature, then rinsed in PBS for 2x10 minutes. After a final washing with cold DPBS (2x250 uL), they were mounted as described above.

### RT-PCR Analysis

Total RNA was extracted from rat E13 or E14 ventral mesencephalic tissue or from 1x10⁹ astrocytes or from 1x10⁹ VMCL-1 cells using RNA-STAT reagent (TelTest, University of Maryland, Baltimore, Md.). First strand cDNA was generated from RNA and amplified by polymerase chain reaction using the manufacturer's procedures.

Reaction products were resolved by 2% agarose gel electrophoresis to determine size and relative abundance of fragments. PCR results for b-actin and GAPDH were included as controls to confirm equal loading of cDNA.

### Chromosomal Analysis

The cells were grown in DMEM/F-12 1:1 medium supplemented with 2.5% FBS, D-glucose (2.5 g/L) and ITS supplement, diluted 1:100. Twenty-four hours later, subcultures at metaphase stage were arrested with colchicine (10 .mu.g/mL). The cells were trypsinized and subjected to hypotonic shock (75 mM KCl). The cells were then fixed in three changes of MeOH/CH₃COOH, 3:1, and air-dried. The cells were then stained using 4% Geisma, and microscopically examined.

### Example 14: 6-HYDROXYDOPAMINE (6-OHDA) MODEL OF PARKINSON'S DISEASE

MANF was next tested in the rodent model of Parkinson's disease, in which the dopaminergic neurons on one side of the brain are lesioned by a neurotoxin, 6-hydroxydopamine (6-OHDA), that is injected close to dopaminergic neurons in the substantia nigra, and selectively taken up into dopaminergic neurons via the dopamine transporter (DAT). 6-OHDA selectively destroys dopaminergic neurons on the injected side of the brain. At biweekly intervals, the experimental animals are challenged by a systemic injection of D-amphetamine or apomorphine, both of which cause the release of DA in the brain. More DA is released on the intact side of the brain. This imbalance in the release of DA causes the animal to rotate to the intact side of the brain (Mendez and Finn, 1975). The behavioural effect can be dramatic. An animal can sometimes rotate at high speed, in a tight circle, the equivalent of chasing its tail. The number of rotations per minute indicates the imbalance in the release of DA, and in turn the extent of the lesion on the experimental side. Drugs like MANF, CDNF and GDNF that protect and induce the regeneration of dopaminergic neurons, decrease the number of rotations per minute, an indication of the restoration of DA release on the lesioned side of the brain. Increased release of DA is, in turn, indicative of regeneration of DA neurons on the lesioned side of the brain.

A MANF homologue, MANF2 (also called Conserved Dopaminergic Neurotrophic Factor or CDNF) was discovered at the University of Helsinki after the publication of MANF.

### Expression of MANF in the Ventral Mesencephalon of the Adult Brain

The expression of MANF was determined in 10 regions in the adult mammalian brain using quantitative RT-PCR: Olfactory bulb, Hippocampus, Cerebral cortex, Striatum, Thalamus, Ventral mesencephalon, Colliculus, Pons, Medulla and Cerebellum. The highest level of expression of MANF was found in the ventral mesencephalon, the same region of the brain where the DA neurons that die in PD are located. This result demonstrates that MANF can be a physiological neurotrophic factor in the adult human brain.

### MANF Releases GABA in the Substantia Nigra

Miniature inhibitory postsynaptic currents (mIPSCs) were recorded from dopaminergic neurons in the substantia nigra. The mIPSCs were completely blocked by bicuculine (10 µM) indicating that they were mediated via a GABA-A receptor mechanism. MANF (5 ng/ml) caused a reversible increase in the frequency of the mIPSCs (Zhou et al, 2006), suggesting that MANF increases the release of GABA from from presynaptic GABAergic terminals in the substantia nigra that synapse with DA neurons.

One of the proposed, contributing causes of Parkinson's disease is the excitotoxic action of glutamate on dopaminergic neurons in the substantia nigra. Furthermore, mGluRs have been implicated in the functioning of dopaminergic neurons and the actions of neuroprotective drugs in the brain. These results demonstrate that a second target for MANF in the treatment of Parkinson's disease is the presynaptic GABAergic terminals in the substantia nigra, mediated by the release of the inhibitory transmitter GABA, that antagonizes the excitotoxic action of glutamate on dopaminergic neurons in the substantia nigra.

### Deposit

Applicant has made a deposit of at least 25 vials containing cell line VMCL-1 with the American Type Culture Collection, Manassas Va., 20110 U.S.A., ATCC Deposit No. PTA-2479. The cells were deposited with the ATCC on Sep. 18, 2000. This deposit of VMCL-1 will be maintained in the ATCC depository, which is a public depository, for a period of 30 years, or 5 years after the most recent request, or for the effective life of the patent, whichever is longer, and will be replaced if it becomes nonviable during that period. Additionally, Applicant has satisfied all the requirements of 37 C.F.R. §§1.801-1.809, including providing an indication of the viability of the sample. Applicant imposes no restrictions on the availability of the deposited material from the ATCC. Applicant has no authority, however, to waive any restrictions imposed by law on the transfer of biological material or its transportation in commerce. Applicant does not waive any infringement of its rights granted under this patent. Other Embodiments All publications and patents mentioned in the above specification are herein incorporated by reference. Various modifications and variations of the described method and system of the invention will be apparent to those skilled in the art without departing from the scope and spirit of the invention.

### Example 15: NEURAL TRANSPLANTATION IN A RAT MODEL OF PARKINSON'S DISEASE (PD)

In this example, MANF molecules are tested in a rat model of Parkinson's Disease (PD). Unilateral 6-OHDA lesions are created in a rat population, as described in Example 14. Within three weeks of the denervation procedure, a standard behavioural test will be used to determine the number of rats that were successfully lesioned.

E14 nigral tissue is harvested for transplantation from timed-pregnant rats, using standardized dissection techniques and procedures. The percent viability of the cells in the nigral tissue will be tested at the following times: 1. Immediately after the dissection and 2. Immediately before transplantation. Viability testing will be preformed on dispersed cells, using the Live Cell - Dead Cell kit from Molecular Probes (Cat. No. L-3224). An estimate of the percentage of dopaminergic neurons in the nigral tissue used for transplantation will be made.

The denervated rats will be divided into three groups (N ≥ 6). Equal amounts of E14 nigral tissue will be primed in the solutions containing different amounts of MANF, for a predetermined period of time (Sortwell et al, 2000). Each vial will contain 500 µl of the compounds to be tested. The experiments will be performed within 7 days of the receipt of the test materials. The coded vials will be kept in the refrigerator, wrapped in foil, at about 4 °C, until used. Following priming, the cells will be transplanted into the denervated neostriatum.

Over a period of 4-8 weeks after the neural transplantation phase of the work, the animals will be tested for functional, behavioural recovery. The animals will then be sacrificed, and the following data derived for the three experimental groups: 1. Percentage survival of transplanted neurons; 2. Percentage survival of transplanted dopaminergic neurons; and 3. Degree of development of transplanted, dopaminergic neurons, assessed by: a) Size (diameter) of dopaminergic neurons and b) Neurite outgrowth.

### Example 16: MANF EXPRESSION IN ADULT MOUSE BY RT-PCR

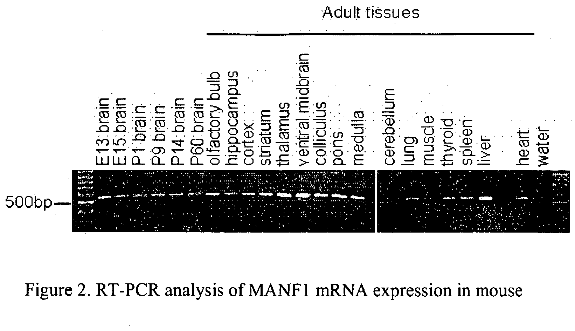

MANF expression in mouse tissue, including mouse brain, was analyzed in different anatomical structures, as well as different points in development. MANF expression was analyzed by quantitative RT-PCR using primers amplifying a fragment of MANF. Standard protocols well known in the art were used, including standards for PCR cycles and temperature. Primers were generated in order to amplify a portion of MANF to yield a band above 500 bp in length. The results are shown in Figure 2. The highest level of expression of MANF in the adult mouse brain was in the substantia nigra pars compacta (SNc). This is the site of localization of DA neurons in the brain.

## Claims

1. A composition comprising mensencephalic astrocyte-derived neurotrophic factor (MANF), for use in treating Parkinson's Disease, wherein MANF is administered to the substantia nigra region of the brain in a subject.

2. A composition comprising MANF for use in treating Parkinson's Disease, wherein MANF is administered to the ventral mesencephalon region in a subject.

3. The composition for use according to claim 1 or 2, wherein a neural stem cell is transplanted to said region.

## Patentansprüche

1. Zusammensetzung, umfassend aus mesencephalischen Astrozyten abgeleiteter neurotropher Faktor (MANF), zur Verwendung beim Behandeln von Parkinson-Krankheit, wobei MANF dem Substantia-nigra-Bereich des Gehirns in einem Subjekt verabreicht wird.

2. Zusammensetzung, umfassend MANF, zur Verwendung beim Behandeln von Parkinson-Krankheit, wobei MANF dem ventralen Mesencephalon-Bereich in einem Subjekt verabreicht wird.

3. Zusammensetzung zur Verwendung nach Anspruch 1 oder 2, wobei eine neurale Stammzelle dem Bereich transplantiert wird.

## Revendications

1. Composition comprenant un facteur neurotrophique dérivé des astrocytes (MANF), destinée à une utilisation dans le traitement de la maladie de Parkinson, la MANF étant administrée à la région de substance noire du cerveau chez un sujet.

2. Composition comprenant la MANF destinée à une utilisation dans le traitement de la maladie de Parkinson, la MANF étant administrée à la région mésencéphale ventrale chez un sujet.

3. Composition destinée à une utilisation selon la revendication 1 ou 2, dans laquelle une cellule souche neuronale est transplantée dans ladite région.
